(19) Europäisches Patentamt
European Patent Office
Office européen des brevets

(11) EP 4 191 245 A2

(12) **EUROPEAN PATENT APPLICATION**

(43) Date of publication:
**07.06.2023 Bulletin 2023/23**

(21) Application number: **22214476.8**

(22) Date of filing: **19.04.2013**

(51) International Patent Classification (IPC):
***G01N 33/50*** *(2006.01)* ***C12Q 1/68*** *(2018.01)*
***G01N 33/569*** *(2006.01)*

(52) Cooperative Patent Classification (CPC):
**C07D 205/04; A61P 25/00; A61P 25/28; A61P 37/02; A61P 37/06; A61P 43/00; C12Q 1/6883; G01N 33/5094; G01N 33/56972;**
C12Q 2600/106; G01N 2800/52

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.04.2013 US 201361808406 P**
**12.04.2013 US 201361811321 P**
**18.04.2013 US 201361813380 P**

(62) Document number(s) of the earlier application(s) in accordance with Art. 76 EPC:
**19204477.4 / 3 647 783**
**17157993.1 / 3 199 947**
**13717513.9 / 2 981 819**

(27) Previously filed application:
**19.04.2013 EP 13058226**

(71) Applicant: **Novartis AG**
**4056 Basel (CH)**

(72) Inventors:
- **BORELL, Hubert**
**4002 Basel (CH)**
- **GARDIN, Anne**
**4002 Basel (CH)**
- **JIN, Yi**
**4002 Basel (CH)**
- **LEGANGNEUX, Eric**
**4002 Basel (CH)**
- **UFER, Mike**
**4002 Basel (CH)**

(74) Representative: **Didelon, Frédéric**
**Novartis Pharma AG**
**Patent Department**
**Lichtstrasse 35**
**4056 Basel (CH)**

Remarks:
This application was filed on 19-12-2022 as a divisional application to the application mentioned under INID code 62.

(54) **IDENTIFYING PATIENT RESPONSE TO S1P RECEPTOR MODULATOR ADMINISTRATION**

(57) The invention provides a method of assessing the appropriate therapeutic dose of 1-{4-[1-(4-cyclohexyl-3-trifluoromethyl-benzyloxyimino)-ethyl]-2-ethyl-benzyl}-azetidine-3-carboxylic acid to administer to a patient in need thereof, comprising the steps of:
(i) testing whether or not the patient has the poor metabolizer genotype; and
(ii) if the patient does not have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose; and
(iii) if the patient does have the poor metaboliser genotype, either
(a) administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at a therapeutic dose below that of the standard therapeutic dose; or
(b) not administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient.

EP 4 191 245 A2

**Figure 1-1 Time-dependent biotransformation of [$^{14}$C]BAF312**

The kinetics of the biotransformation of 1 and 10 µM [$^{14}$C]BAF312 was investigated using human liver microsomes (0.3 mg protein/mL). The disappearance of [$^{14}$C]BAF312 was determined by HPLC analysis combined with radiodetection.

1 µM [$^{14}$C]BAF312

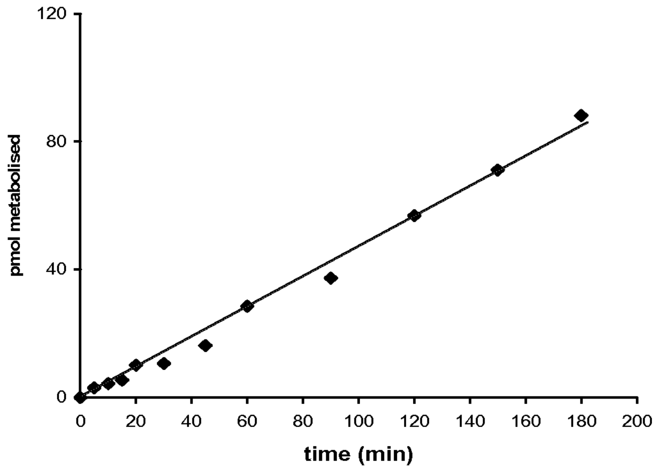


10 µM [$^{14}$C]BAF312

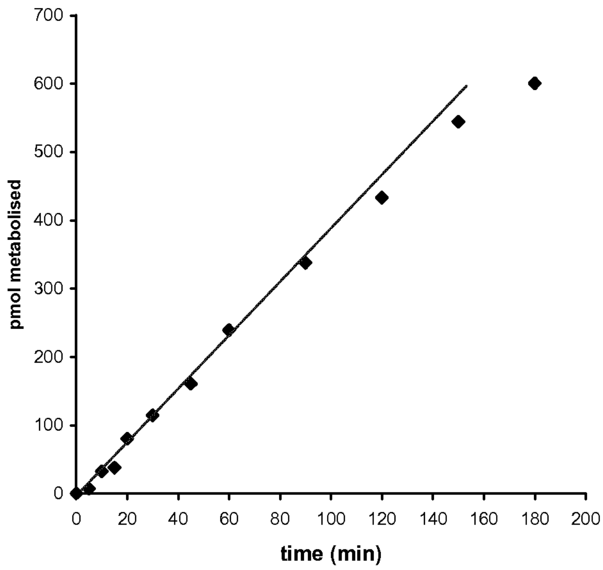




(Cont. next page)

**Figure 1-2 Protein-dependent biotransformation of [$^{14}$C]BAF312**

The protein-dependence of 10 µM [$^{14}$C]BAF312 metabolism was investigated (60 minutes incubations) using human liver microsomes. The disappearance of [$^{14}$C]BAF312 was determined by HPLC analysis combined with radiodetection.

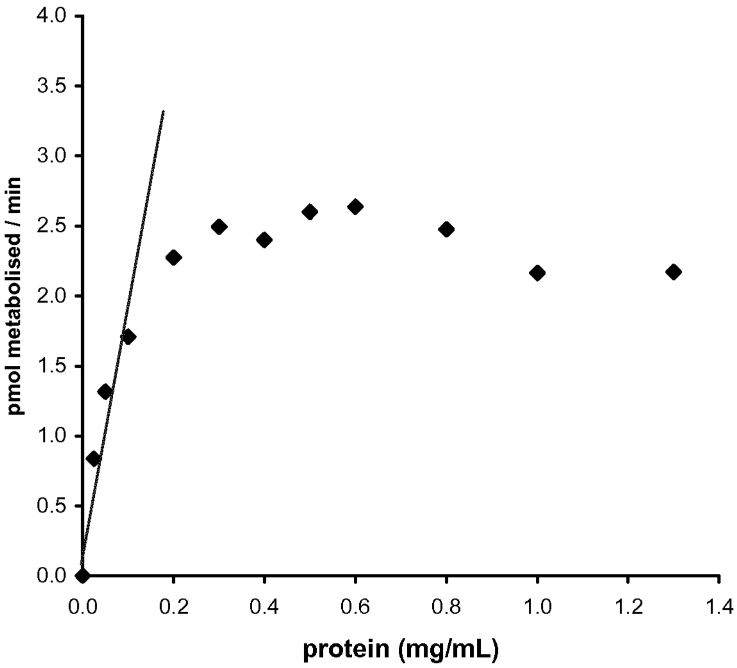




(Cont. next page)

**Figure 1-3 Enzyme kinetics of [$^{14}$C]BAF312 biotransformation in human liver microsomes**

The concentration-dependent kinetics of [$^{14}$C]BAF312 biotransformation in pooled human liver microsomes (0.1 mg/mL) after 90 minutes incubation was plotted as Michaelis-Menten plot (upper) and as Eadie-Hofstee plot (bottom) fitted with substrate inhibition model.

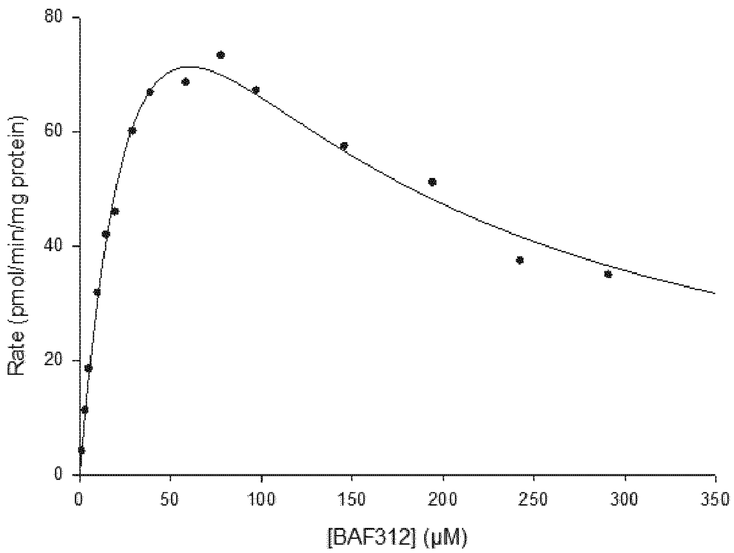


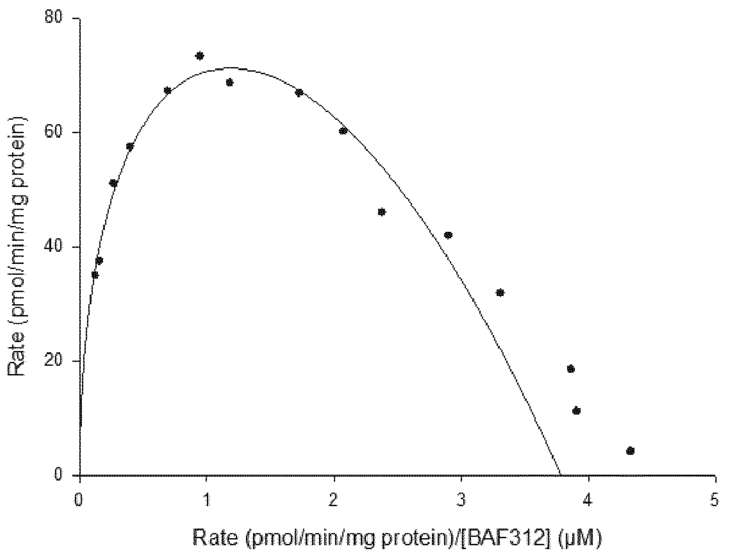


| Kinetic model | $V_{max}$ [pmol/min/mg] | $K_m^{app}$ [µM] | $V_{max}/K_m$ µL/mg/min |
|---|---|---|---|
| Substrate Inhibition (uncompetitive) | 191 ± 25 | 50.3 ± 9.7 | 3.8 |



(Cont. next page)

**Figure 1-4 Biotransformation of [$^{14}$C]BAF312 by recombinant human CYP P450s**

The biotransformation of [$^{14}$C]BAF312 by recombinant enzymes (30 pmol/mL) in insect cell membranes expressing a single human cytochrome P450 isoenzyme and by HLM (108 pmol CYP/mL) was investigated after 30 minutes incubations at 10 and 40 µM. The formation of metabolites was determined by HPLC analysis combined with radiodetection.

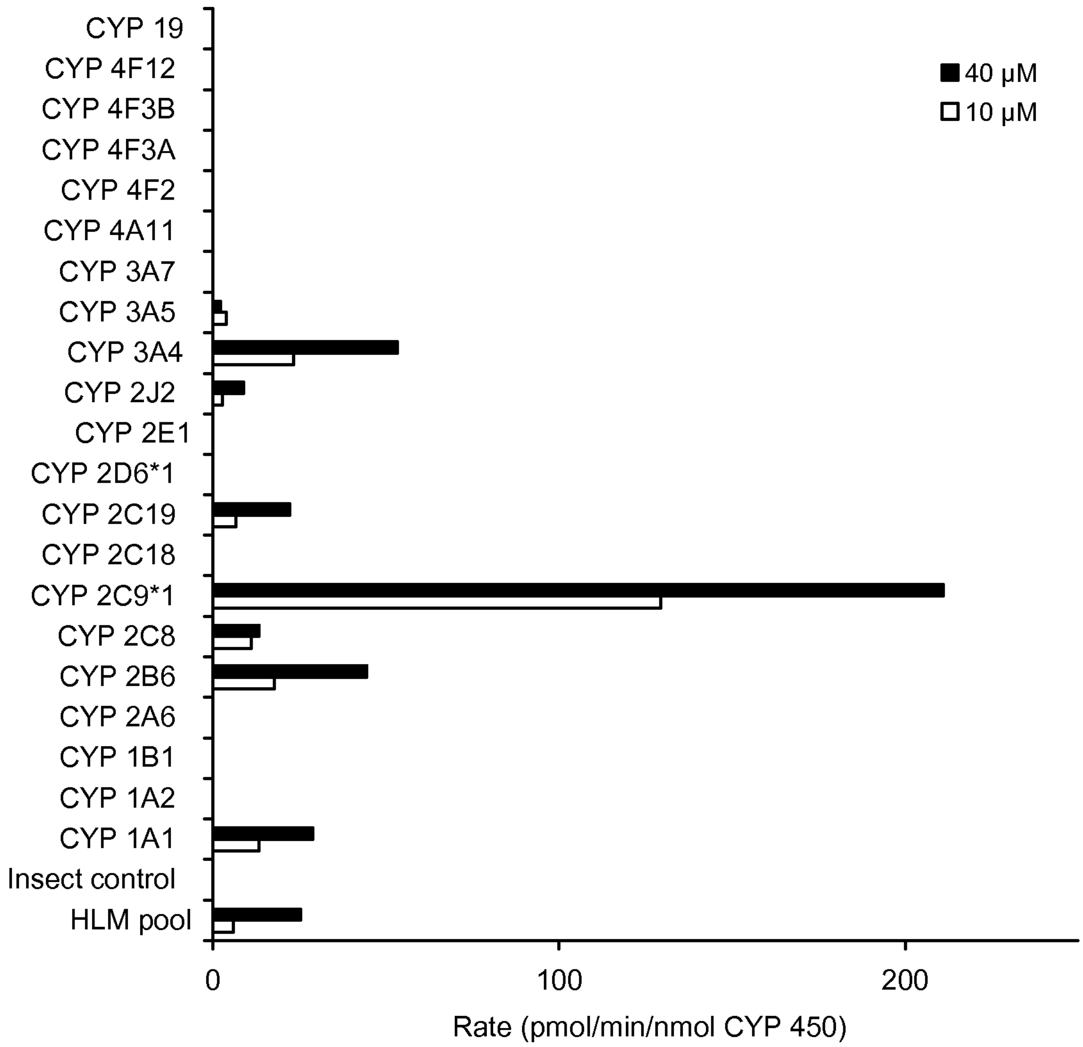




(Cont. next page)

**Figure 1-5 Enzyme kinetics of [$^{14}$C]BAF312 metabolism by rhCYP2C9**

The concentration-dependent kinetics of [$^{14}$C]BAF312 biotransformation (total metabolites) in rhCYP2C9 (50 pmol/mL) after 60 minutes incubation was plotted as Michaelis-Menten plot (upper) and as Eadie-Hofstee plot (V vs. V/S, bottom) fitted with substrate inhibition model using substrate concentrations 5-300 µM.

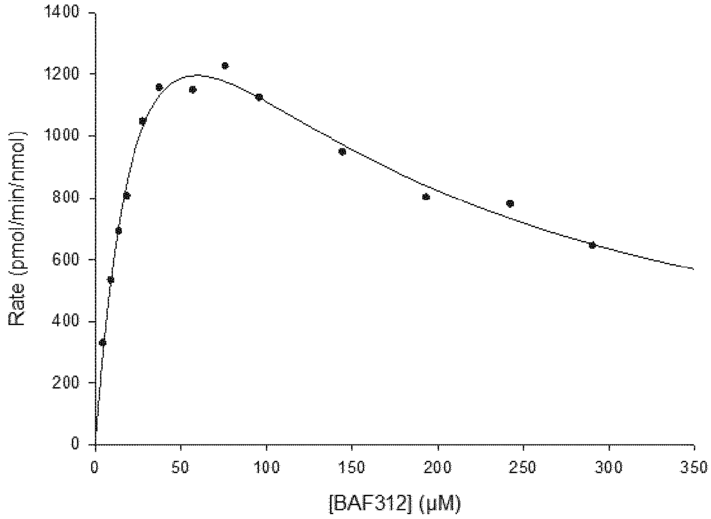


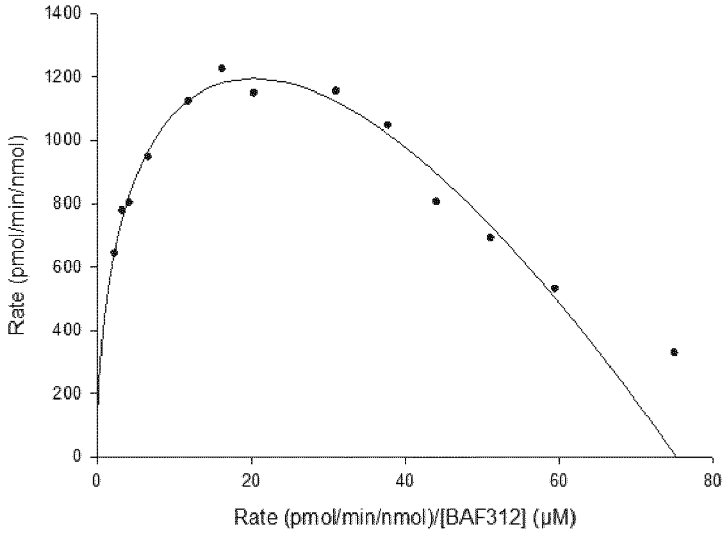


| $V_{max}$ [pmol/min/nmol] | $K_m^{app}$ [µM] | $V_{max}/K_m$ µL/nmol/min |
|---|---|---|
| 2596 ± 233 | 34.5 ± 5.0 | 75.2 |



(Cont. next page)

**Figure 1-6 Enzyme kinetics of [$^{14}$C]BAF312 metabolism by rhCYP3A4**

The concentration-dependent kinetics of [$^{14}$C]BAF312 biotransformation (total metabolites) in rhCYP3A4 (50 pmol/mL) after 30 minutes incubation was plotted as Michaelis-Menten plot (upper) and as Eadie-Hofstee plot (V vs. V/S, bottom) using substrate concentrations 5-250 µM.

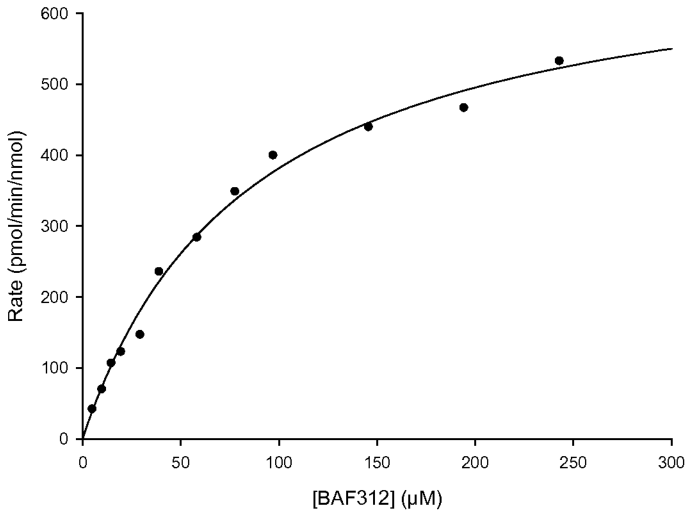


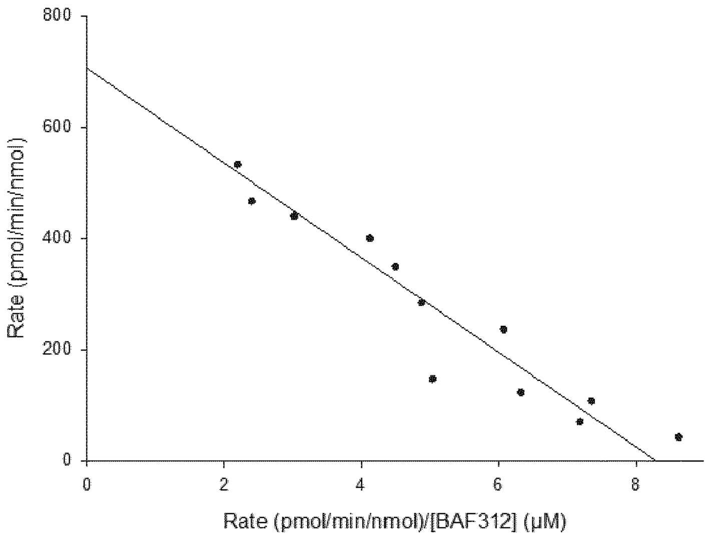


| $V_{max}$ [pmol/min/nmol] | $K_m^{app}$ [µM] | $V_{max}/K_m$ µL/nmol/min |
|---|---|---|
| 706 ± 31 | 85.1 ± 8.6 | 8.30 |



(Cont. next page)

**Figure 1-7 Inhibition of BAF312 metabolism in HLM by chemical inhibitors**

Biotransformation of [$^{14}$C]BAF312 (5 µmol/L) by human liver microsomes (0.1 mg/mL) was investigated in the presence and absence of different inhibitors. The supernatants of incubations after 90 minutes were analyzed by HPLC with radiodetection.

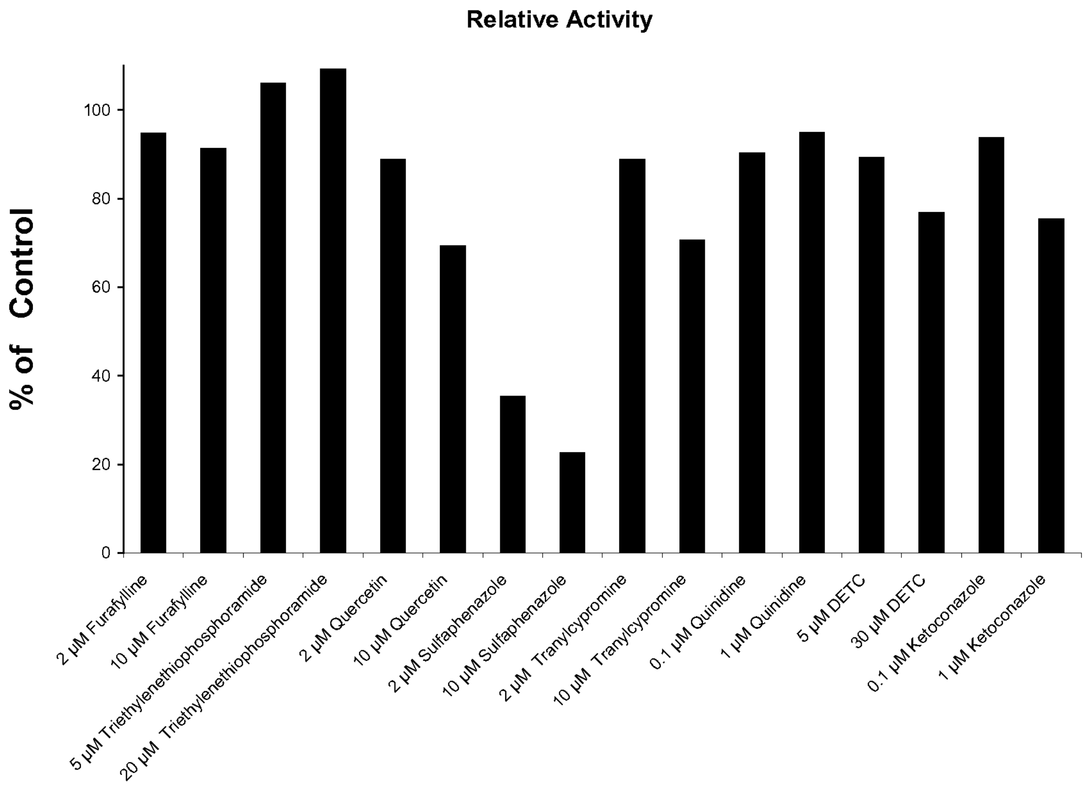




(Cont. next page)

**Figure 1-8 Comparison of [$^{14}$C]BAF312 metabolism rates in HLM from individual donors with 3 different CYP2C9 genotypes (Data from Table 1-5)**

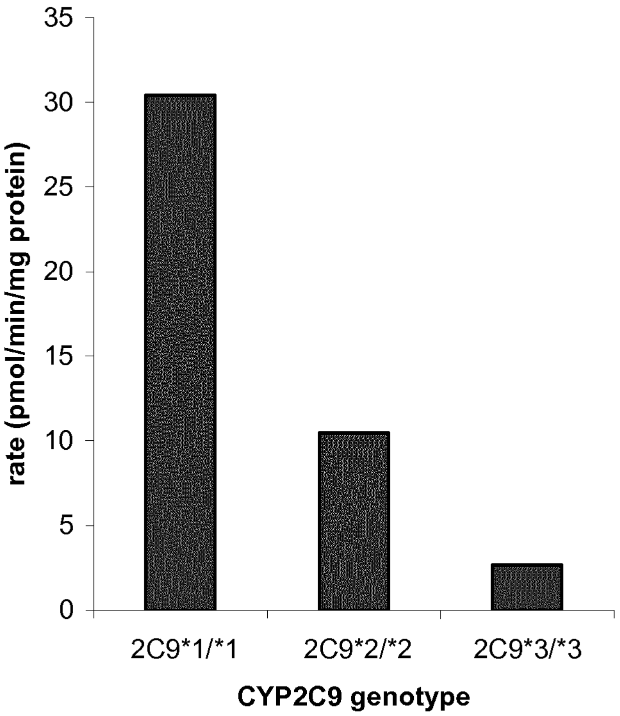

## Description

### Field of the Invention

The present invention relates to a method of determining the likely response of patients to administration of the S1P receptor modulator BAF312 (1-{4-[1-(4-cyclohexyl-3-trifluoromethyl-benzyloxyimino)-ethyl]-2-ethyl-benzyl} -azetidine-3-carboxylic acid). More specifically, the invention relates to identifying and selecting patients who may benefit from either a modified dosage regimen of BAF312 to that of other patients or identifying patients who may be less suited to treatment with BAF312. In addition, the invention relates to a method of determining when administration of drugs considered incompatible with BAF312 may be initiated following the end of a treatment period with BAF312.

### Background

BAF312 belongs to the class of S1P receptor modulators. These are compounds which signal as agonists at one or more sphingosine-1 phosphate receptors, for example, S1P1 to S1P8. The binding of an agonist to a S1P receptor may, for example, result in the dissociation of intracellular heterotrimeric G-proteins into Gα-GTP and Gβγ-GTP, and/or the increased phosphorylation of the agonist-occupied receptor, and/or the activation of downstream signaling pathways/kinases.

S1P receptor modulators or agonists are useful therapeutic compounds for the treatment of various conditions in mammals, especially in human beings. For example, the efficacy of S1P receptor modulators or agonists in the prevention of transplant rejection has been demonstrated in rat (skin, heart, liver, small bowel), dog (kidney), and monkey (kidney) models. In addition, due to their immune-modulating potency, S1P receptor modulators or agonists are also useful for the treatment of inflammatory and autoimmune diseases.

BAF312 is generally well tolerated in human patients but like some other S1P receptor modulators produces a negative chronotropic effect when first administered, the extent of which increases with increasing dose. In the case of BAF312, the negative chronotropic effect can be ameliorated by the use of a dose titration regimen as described in WO2010/072703.

However, the effect of a given dose of BAF312 (including side effects such as the negative chronotropic effect or the duration of the washout period) may be different in some patients than in others, for example depending on how extensively the patient metabolises BAF312. It would therefore be desirable, in order to improve the risk benefit ratio for these patients, to be able to identify patients for whom these effects would be significantly different and adjust the treatment regimen accordingly.

### Brief Disclosure of the Invention

In a first aspect of the invention, there is provided a method of assessing the appropriate therapeutic dose of 1-{4-[1-(4-cyclohexyl-3-trifluoromethyl-benzyloxyimino)-ethyl]-2-ethyl-benzyl}-azetidine-3-carboxylic acid to administer to a patient in need thereof, comprising the steps of:

(i) testing whether or not the patient has the poor metabolizer genotype; and
(ii) if the patient does not have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose; or
(iii) if the patient does have the poor metaboliser genotype, either

(a) administering 1 -{4-[1 -(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at a therapeutic dose below that of the standard therapeutic dose; or
(b) not administering 1-{4-[1-(4-cyclohexyl-3-trifluoromethyl-benzyloxyimino)-ethyl]-2-ethyl-benzyl}-azetidine-3-carboxylic acid to the patient.

In a second aspect of the invention, there is provided a method of initiating use of a drug recommended not to be used with 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, comprising the steps of:

(i) testing whether or not the patient has the poor metaboliser genotype; and
(ii) if the patient does not have the poor metaboliser genotype, inititiating use of the drug within a set period after the last dose of 1-{4-[1-(4-cyclohexyl-3-trifluoromethyl-benzyloxyimino)-ethyl]-2-ethyl-benzyl}-azetidine-3-carboxylic acid has been administered to the patient; or

(iii) if the patient does have the poor metaboliser genotype genotype, initiating use of the drug within a period longer than the set period of step (ii) above after the last dose of 1-{4-[1-(4-cyclohexyl-3-trifluoromethyl-benzyloxyimino)-ethyl]-2-ethyl-benzyl}-azetidine-3-carboxylic acid has been administered to the patient.

In a third aspect of the invention, there is provided a method of treating a patient in need with 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, comprising the steps of:

(i) testing whether or not the patient has the poor metabolizer genotype; and

(ii) if the patient does not have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose; or

(iii) if the patient does have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose in combination with a CYP2C9 metabolic activity promoter.

In a fourth aspect of the invention, there is provided a method for the treatment of an autoimmune condition in a patient in need thereof, said patient having the poor metabolizer genotype, comprising administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient in a daily amount in the range from 0.25 - 0.75mg.

In a fifth aspect of the invention, there is provided 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, for use in a method of treating a patient suffering from an autoimmune condition, said patient having the poor metabolizer genotype, said method comprising administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient in a daily amount in the range from 0.25 - 0.75mg.

In a sixth aspect of the invention, there is provided 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, for use in a method of treating a patient suffering from an autoimmune condition, said method comprising the steps of:

(i) testing whether or not the patient has the poor metabolizer genotype; and

(ii) if the patient does not have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose; or

(iii) if the patient does have the poor metaboliser genotype, either

(a) administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at a therapeutic dose below that of the standard therapeutic dose; or

(b) not administering 1-{4-[1-(4-cyclohexyl-3-trifluoromethyl-benzyloxyimino)-ethyl]-2-ethyl-benzyl}-azetidine-3-carboxylic acid to the patient.

In a seventh aspect of the invention, there is provided 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, for use in a method of treating a patient in need, said method comprising the steps of:

(i) testing whether or not the patient has the poor metabolizer genotype; and

(ii) if the patient does not have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose; or

(iii) if the patient does have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose in combination with a CYP2C9 metabolic activity promoter.

In an eighth aspect of the invention, there is provided a pharmaceutical combination comprising (a) 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof; and (b) a CYP2C9 metabolic activity promoter for simultaneous, separate or sequential use.

In a ninth aspect of the invention, there is provided a method of optimizing the daily dosage of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof to patient in need, said method comprising the steps of:

(i) measuring the patient blood lymphocyte level following administration of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof at the daily therapeutic dosage; and
(ii) in cases where the blood lymphocyte level is reduced below a level considered clinically optimal following daily administration of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof at the therapeutic dosage, reducing the daily dosage to a reduced dosage.

In a tenth aspect of the invention, there is provided 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, for use in a method of treating a patient in need, said method comprising the steps of:

(i) measuring the patient blood lymphocyte level following administration of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof at the daily therapeutic dosage; and
(ii) in cases where the blood lymphocyte level is reduced below a level considered clinically optimal following daily administration of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof at the therapeutic dosage, reducing the daily dosage to a reduced dosage.

In an eleventh aspect of the invention, there is provided a method of treating a patient in need with 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, comprising the steps of:

(i) testing whether or not the patient has the poor metabolizer genotype; and
(ii) if the patient does not have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose with a certain level of patient monitoring by a medical practitioner; or
(iii) if the patient does have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose, with an increased level of patient monitoring by a medical practitioner.

In a twelfth aspect of the invention, there is provided 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, for use in a method of treating an autoimmune condition, said method comprising the steps of:

(i) testing whether or not the patient has the poor metabolizer genotype; and
(ii) if the patient does not have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose with no or a low level of patient monitoring by a medical practitioner; or
(iii) if the patient does have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose, with an increased level of patient monitoring by a medical practitioner.

In a thirteenth aspect of the invention there is provided 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, in the manufacture of a medicament for the treatment of an autoimmune disease, said treatment being by a method according to any of the first to twelfth aspects above.

Further aspects and embodiments are provided in the detailed disclosure of the invention.

## Brief Description of the Figures

**Figure 1-1** shows a time-dependent biotransformation of [$^{14}C$]BAF312

**Figure 1-2** shows a protein-dependent biotransformation of [$^{14}C$]BAF312

**Figure 1-3** shows enzyme kinetics of [$^{14}C$]BAF312 biotransformation in human liver microsomes

**Figure 1-4** shows a biotransformation of [$^{14}C$]BAF312 by recombinant human CYP P450s

**Figure 1-5** shows enzyme kinetics of [$^{14}C$]BAF312 metabolism by rhCYP2C9

**Figure 1-6** shows enzyme kinetics of [$^{14}C$]BAF312 metabolism by rhCYP3A4

**Figure 1-7** shows inhibition of BAF312 metabolism in HLM by chemical inhibitors

**Figure 1-8** shows a comparison of [$^{14}C$]BAF312 metabolism rates in HLM from individual donors with 3 different CYP2C9 genotypes

## Detailed Disclosure of the invention

For the avoidance of doubt, it is hereby stated that the information disclosed earlier in this specification under the heading "Background" is relevant to the invention and is to be read as part of the disclosure of the invention.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification (which term encompasses both the description and the claims) is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The term "treatment" includes: (1) preventing or delaying the appearance of clinical symptoms of the state, disorder or condition developing in an animal, particularly a mammal and especially a human, that may be afflicted with or predisposed to the state, disorder or condition but does not yet experience or display clinical or subclinical symptoms of the state, disorder or condition; (2) inhibiting the state, disorder or condition (e.g. arresting, reducing or delaying the development of the disease, or a relapse thereof in case of maintenance treatment, of at least one clinical or subclinical symptom thereof); and/or (3) relieving the condition (i.e. causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

Generally, the term "prodrug" refers to a compound that is administered as an inactive or less than fully active chemical derivative that is subsequently, preferably within the human body, converted to an active pharmacological agent. Prodrugs include drugs having a functional group which has been transformed into a reversible derivative thereof. Typically, such prodrugs are transformed into the active drug by hydrolysis. For example, reversible derivatives of a carboxylic acid may be an ester, including e.g. an alkyl and acyloxyalkyl ester, or an amide. Reversible derivatives of amines may be amides, carbamates, imines or enamines. In some cases, the prodrug may also be a salt form. Prodrugs also include compounds convertible to the active drug by an oxidative or reductive reaction. Examples comprise N- and O-dealkylation, oxidative deamination, N-oxidation, epoxidation, azo reduction, sulfoxide reduction, disulfide reduction, bioreductive alkylation, and nitro reduction. "BAF312" as used herein is understood to comprise the compound of formula

(I) as well as the pharmaceutically acceptable salts, solvates, hydrates and/or prodrugs thereof.

The term "therapeutic dosage" means the daily maintenance dose of the drug which is administered to patients for treatment or prevention of the disease to be treated or prevented. This dosage may be selected to give the optimum balance of efficacy vs safety.

The therapeutic dosage may be administered at the start of the treatment period or following a titration regimen in which the dosage is increased from a level below the therapeutic dosage up to the therapeutic dose.

The term "poor metaboliser genotype" includes patients who experience a significantly higher exposure following BAF312 administration than normal patients at a given drug dose e.g. 2mg once daily of BAF312. The poor metabolizer genotype may include the subtype(s) of the CYP2C9 genotype associated with poor metabolism of 1-{4-[1-(4-cyclohexyl-3-trifluoromethyl-benzyloxyimino)-ethyl]-2-ethyl-benzyl}-azetidine-3-carboxylic acid. The poor metabolizer genotype includes the CYP2C9*3*3 and CYP2C9*2*3 genotypes, for example the CYP2C9*3*3 genotype.

In a preferred embodiment, the poor metaboliser genotype is the CYP2C9*3*3 genotype.

The term "standard therapeutic dosage" means the therapeutic daily dosage for patients who do not have the poor metabolizer genotype. The standard therapeutic dose may be greater than or equal to 0.2 mg, for example greater than or equal to 0.4 mg, greater than or equal to 1.0 mg or greater than or equal to 1.5mg. The standard therapeutic dose may be less than or equal to 8mg, for example less than or equal to 5mg, less then or equal to 4mg or less than or equal to 2.5mg.

In a preferred embodiment, the standard therapeutic dose is in the range from 1.5 to 2.5 mg, for example about 2mg per day.

In the case of patients having the poor metaboliser genotype, the daily therapeutic dosage may be lower then the standard therapeutic dosage. For example the therapeutic dosage for this class of patients may be greater than or equal to 0.1mg, such as greater than or equal to 0.25mg or greater than or equal to 0.4mg. The therapeutic dosage may be less than or equal to 1mg such as less than or equal to 0.9mg or less than or equal to 0.75mg.

In a preferred embodiment, in the case of patients having the poor metabolizer genotype, the daily therapeutic dosage may be in the range from 0.25-0.75mg, for example 0.25mg, 0.5mg or 0.75mg, preferably 0.5mg.

As used herein, an "amount", "dose" or "dosage" of BAF312 as measured in milligrams refers to the milligrams of BAF312 (free form) present in a preparation, regardless of the form of the preparation. A "dose of 2 mg BAF312" means the amount of BAF312 (free form) in a preparation is 2 mg, regardless of the form of the preparation. Thus, when in the form of a salt, e.g. the BAF312 hemifumarate salt, the weight of the salt form necessary to provide a dose of 2 mg BAF312 would be greater than 2 mg due to the presence of the additional hemifumarate ion.

Patients in need of treatment with BAF312 include patients suffering from chronic longterm diseases, such as autoimmune diseases, e.g. multiple sclerosis, polymyositis, dermatomyositis, lupus nephritis, rheumatoid arthritis, inflammatory bowel diseases or psoriasis. In an embodiment of the invention, patients in need of treatment are patients suffering from multiple sclerosis, for example relapsing multiple sclerosis (RMS), relapsing remitting multiple sclerosis (RRMS), primary progressive multiple sclerosis (PPMS), secondary progressive multiple sclerosis with relapses (rSPMS), secondary progressive multiple sclerosis without relapses (SPMS) e.g. for patients suffering from rSPMS or SPMS.

In a preferred embodiment, the patient is suffering from multiple sclerosis, polymyositis or dermatomyositis.

In a preferred embodiment, the patient is suffering from multiple sclerosis e.g. secondary progressive multiple sclerosis with relapses (rSPMS) or secondary progressive multiple sclerosis without relapses (SPMS).

In an aspect, where the patient is identified as being a poor metabolizer type, the patient may not be administered 1-{4-[1-(4-cyclohexyl-3-trifluoromethyl-benzyloxyimino)-ethyl]-2-ethyl-benzyl}-azetidine-3-carboxylic acid if the patient is considered to belong to a higher risk category. For example, 1-{4-[1-(4-cyclohexyl-3-trifluoromethyl-benzyloxyimino)-ethyl]-2-ethyl-benzyl}-azetidine-3-carboxylic acid may not be administered to poor metabolizer patents at risk of cardiac side effects, for example patients at risk of heart failure, arrythmias, patients with high grade atrio-ventricular blocks or sick sinus syndrome, patients with a history of syncopal episodes, or patients requiring or under beta blockers, or patients requiring or under anti-arrhythmic treatment, such as patients under treatment with Class Ia (e.g. quinidine, procainamide) or Class III anti-arrhythmic drugs (e.g., amiodarone, sotalol).

In the second aspect of the invention, the set period of step (ii) within which the patient who is not a poor metabolizer may initiate use of the incompatible drug, will depend on the degree of incompatibility of the dug with BAF312. The period of step (ii) may be a period greater than or equal to 3 or 4 days, for example greater than or equal to 5 days, for example greater than or equal to 6 days or 7 days following administration of the last dose of BAF312. The set period of step (ii) may also be greater than 10 days, for example greater than 12 days or greater than 14 days.

The set period of step (ii) may also be less than 21 days, for example less than 14 days or less than 10, 8 or 7 days.

In an aspect, the set period of step (ii) may be in the range from 3-14 days, for example 4-12 days or 5-10 days e.g 6, 7 or 8 days.

As mentioned in the second aspect, in step (iii) the patient who is a poor metabolizer may initiate use of the incompatible drug, within a period greater than the patient who is not a poor metabolizer. For example, the period of step (iii) may be a period greater than or equal to 14 days, for example greater than or equal to 21 days, for example

greater than or equal to 28 day, 35 days or 42 days following administration of the last dose of BAF312.

The set period of step (iii) may also be less than 56 days, for example less than 49 or 42 days.

In an aspect, the set period of step (iii) may be in the range from 14-63 days, for example 14-56 days or 21-49 days or 28-42 days.

In the second aspect, or any related aspect, the incompatible drug may be any drug which could be classified as not recommended for administration with BAF312 e.g. a drug which, when administered with BAF312, could potentially cause an increased risk of an adverse effect in the patient or reduced efficacy of either BAF312 or the incompatible drug.

For example, the incompatible drug may be a drug sometimes described as prolonging the QT interval, for example carbamazepine. Alternatively, the incompatible drug may be one or more of Amphetamine, Phentermine, Metaproterenol, Clomipramine, Dolasetron, Chloroquine, Moxifloxacin, Diphenhydramine, Sotalol, Clarithromycin, Terbutaline, Ephedrine, Epinephrine, Vandetanib, Nicardipine, Quinidine, Citalopram, Fosphenytoin, Escitalopram, Ciprofloxacin, Clozapine, Cocaine, Methylphenidate, Amiodarone, Ibutilide, Perflutren lipid microspheres, Trazodone, Tolterodine, Amphetamine, Fluconazole or Dobutamine, Methadone, Isradipine, Erythromycin, Venlafaxine, Amitriptyline, Erythromycin, Lithium, Artenimol+piperaquine, Gemifloxacin, Iloperidone, Phentermine, Felbamate, Ofloxacin, Dexmethylphenidate, Foscarnet, Ziprasidone, Eribulin, Haloperidol, Halofantrine, Astemizole, Droperidol, Dopamine, Paliperidone, Isoproterenol, Telithromycin, Granisetron, Levofloxacin, Vardenafil, Norepinephrine, Probucol, Indapamide, Isoproterenol, Thioridazine, Sibutramine, Metaproterenol, Methadone, Domperidone, Dronedarone, Pentamidine, Phenylephrine, Ketoconazole, Chloral hydrate, Tamoxifen, Imipramine, Disopyramide, Ritonavir, Diphenhydramine, Pimozide, Levomethadyl, Nortriptyline, Paroxetine, Pseudoephedrine, Pentamidine, Famotidine, Desipramine, Oxytocin, Fenfluramine, Epinephrine, Midodrine, Procainamide, Tacrolimus, Procainamide, Cisapride, Albuterol, Fluoxetine, Quinine sulfate, Quinidine, Ranolazine, Mirtazapine, Galantamine, Ranolazine, Mirtazapine, Galantamine, Atazanavir, Risperidone, Methylphenidate, Roxithromycin, Ephedrine, Octreotide, Fluoxetine, Terfenadine, Trimethoprim-Sulfa, Sertindole, Mesoridazine, Salmeterol, Sertindole, Doxepin, Bedaquiline, Sevoflurane, Amisulpride, Itraconazole, Atomoxetine, Trimipramine, Sunitinib, Amantadine, Flecainide, Nilotinib, Gatifloxacin, Chlorpromazine, Dofetilide, Arsenic trioxide, Lapatinib, Sevoflurane, Moexipril/HCTZ, Alfuzosin, Bepridil, Solifenacin, Voriconazole, Protriptyline, Lisdexamfetamine, Levalbuterol, Ritodrine, Sparfloxacin, Tizanidine, Azithromycin, Ondansetron, Sertraline or Olanzapine.

In an embodiment, the incompatible drug may be a drug sometimes described as inducing a negative chronotropic effect. In this embodiment, the incompatible drug may be selected from beta blockers e.g. metoprolol, acetylcholine, digoxin or calcium channel blockers e.g. diltiazem and verapamil.

In an embodiment, the incompatible drug may be a drug which is a strong or moderate inhibitor of CYP2C9 activity. In this embodiment, the incompatible drug may be selected from amiodarone, fluconazole, miconazole or oxandrolone.

In an embodiment, for example in the case of patients who are poor metabolizer patients, the incompatible drug may be a strong or moderate CYP3A4 inhibitor. In this embodiment, the incompatible drug may be selected from boceprevir, clarithromycin, conivaptan, grapefruit juice, indinavir, itraconozole, ketoconazole, lopinavir, mibefradil, nefazodone, nelfinavir, posaconazole, ritonavir, squinavir, telaprevir, telithromycon or voriconazole.

In an embodiment, for example in the case of patients who are not poor metabolizer patients, the incompatible drug may be a strong or moderate CYP2C9 inducer. In this embodiment, the incompatible drug may be selected from carbamazepine or rifampin.

A strong inhibitor for a specific CYP is defined as an inhibitor that increases the plasma AUC of a substrate for that CYP by equal to or more than 5-fold or causes a more than 80% decrease in clearance.

A moderate inhibitor for a specific CYP is defined as an inhibitor that increases the plasma AUC of a substrate for that CYP by less than 5-fold but equal to or more than 2-fold or causes 50- 80% decrease in clearance.

## CYP2C9 metabolic activity promoter

In the third and any other relevant aspects (e.g. the seventh and eighth aspects), the CYP2C9 promoter may be any drug which increases the level of activity of CYP2C9 in the poor metabolizer patient, preferably to a level at which the patient metabolises BAF312 to a level comparable with a non-poor metabolizer patient e.g. within 30%, 20% or 10% of the level of the average non-poor metabolizer. The CYP2C9 promoter may also be any drug which increases the level of activity of CYP2C9 in the poor metabolizer patient to a level at which the same therapeutic dosage and/or dosage regimen (e.g. titration scheme) of BAF312 is considered medically appropriate for both poor metabolizer patients and non-poor metaboliser patient.

An example of a CYP2C9 promoter is rifampin or carbamezipine, which may be administered to poor metabolizer patients at a dosage such that CYP2C9 activity of the patient is adjusted to a level comparable to that of non-poor metabolisers e.g. within 30%, 20% or 10% of the level of the average non-poor metabolizer. In an embodiment, rifampin or carbamezipine may be administered to a poor metaboliser patient at a dosage at which the same therapeutic dosage and/or dosage regimen (e.g. titration scheme) of BAF312 is considered medically appropriate for both poor metabolizer

patients and non-poor metaboliser patients.

In aspects where BAF312 is administered in combination with a CYP2C9 metabolic activity promotor, the administration may be separate, sequential or simultaneous.

Simultaneous administration includes administration of BAF312 and the CYP2C9 metabolic activity promotor (e.g. rifampin or carbamezipine) as a fixed dose combination or as two individual formulations. The invention therefore includes a fixed dose combination of BAF312 and a CYP2C9 metabolic activity promotor (e.g. rifampin or carbamezipine).

BAF312 is preferably administered at the standard therapeutic dosage. The CYP2C9 metabolic activity promotor is preferably administered at a dosage suitable to to upregulate CYP2C9 to a level where a reduced dosage of BAF312 is not considered clinically necessary.

1-{4-[1-(4-cyclohexyl-3-trifluoromethyl-benzyloxyimino)-ethyl]-2-ethyl-benzyl]-azetidine-3-carboxylic acid forms

BAF312 (with the INN Siponimod) has the chemical name 1-{4-[1-(4-cyclohexyl-3-trifluoromethyl-benzyloxyimino)-ethyl]-2-ethyl-benzyl}-azetidine-3-carboxylic acid and has the structure of formula (I) below:

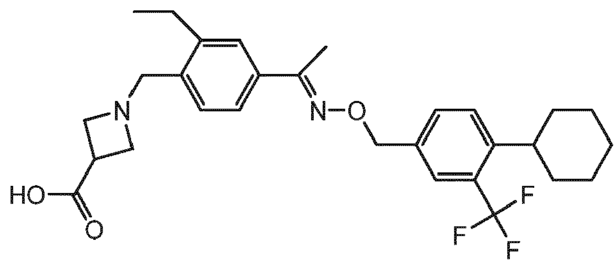

(I)

1-{4-[1-(4-cyclohexyl-3-trifluoromethyl-benzyloxyimino)-ethyl]-2-ethyl-benzyl}-azetidine-3-carboxylic acid may be administered as a free base, as a pharmaceutically acceptable salt (including polymorphic forms of the salt) or as a prodrug.

Pharmaceuticaly acceptable salt forms include hydrochloride, malate, oxalate, tartrate and hemifumarate.

In a preferred aspect, 1-{4-[1-(4-cyclohexyl-3-trifluoromethyl-benzyloxyimino)-ethyl]-2-ethyl-benzyl}-azetidine-3-carboxylic acid is administered as a hemifumarate salt.

Prodrug forms of 1-{4-[1-(4-cyclohexyl-3-trifluoromethyl-benzyloxyimino)-ethyl]-2-ethyl-benzyl}-azetidine-3-carboxylic acid include forms having a functional group which has been transformed into a reversible derivative thereof. Typically, such prodrugs are transformed to the active drug by hydrolysis. As examples may be mentioned esters of the carboxylic acid group.

Titration Regimens

As previously stated, the therapeutic dosage may be administered at the start of the treatment period or following a titration regimen in which the dosage is increased from a level below the therapeutic dosage up to the therapeutic dose in order to minimize the negative chronotropic effects and/or the heart effects possibly associated with S1P receptor modulator or agonist therapy.

Heart effects include AV blocks, which include first degree AV blocks (e.g. PR intervals greater then 0.2 seconds) and second degree AV blocks e.g. first degree AV blocks. Heart effects include heart pauses e.g. heart pauses greater than 2 seconds.

During the titration regimen the dosage is lower than the therapeutic dosage and is increased, optionally stepwise, until the therapeutic dosage is reached. Thereafter the treatment is preferably continued with the therapeutic dosage.

The duration of the titration regiment is the time period beginning on the day when the drug is first administered until and including the first day at which the drug is administered at the therapeutic dose.

Preferably during the titration regimen, the drug is administered in a dosage regimen such that daily decrease in heart rate (e.g. average or minimum daily heart rate) is acceptable or clinically not significant, or that the sinus rhythm of the patient is normal. For example, the daily decrease in heart rate (e.g. average or minimum daily heart rate) may be less than about 4bpm, e.g. less than about 3 bpm or less than about 2bpm.

The term "normal sinus rhythm" refers to the sinus rhythm of the patient when not undergoing treatment. The evaluation of normal sinus rhythm is within the ability of a physician. A normal sinus rhythm will generally give rise to a heart rate in the range from 60-100 bpm.

Preferably, the dosage of the drug is increased during the titration period stepwise in a defined incremental

ratio up to the therapeutic dosage.

In an embodiment, the daily dosage during the titration period is governed by a Fibonacci series i.e. the dosage given on a specific day is the sum of the dosages on the previous two days. In an aspect of this embodiment, some variation in this scheme is permitted. For example, the dosage on a given day may be the sum of the dosages on the two previous days ± 40%, for example ± 30%, for example ± 20% or ± 10%.

The exact titration regimen will depend on whether the therapeutic dosage to be reached is the standard therapeutic dosage or the lower therapeutic dosage to be administered to poor metabolizer patients.

For example in the case of poor metabolizer patients, where the therapeutic dosage is lower, the titration regimen may be e.g. 5 days or less, 4 or 3 days or less, for example 2 days or less. Generally, the titration stage for poor metabolizer patients will last at least 1 day and may last 2 days or 3 days. In a preferred aspect, the titration stage for poor metabolizer patients lasts 3 or 4 days, for example 3 days.

In an aspect, where the patient is a poor metabolizer patient, and the therapeutic dosage is 0.5mg, the titration regimen may be day 1 - 0.25mg; day 2 - 0.25mg; day 3 - 0.5mg (therapeutic dose).

In an aspect, where the patient is a poor metabolizer patient, and the therapeutic dosage is 0.75mg, the titration regimen may be day 1 - 0.25mg; day 2 - 0.25mg; day 3 - 0.5mg; day 4 - 0.75mg (therapeutic dose).

In the case of patients that are not poor metabolisers, the titration stage may be 4 days, 5 days, 6 days or 7 days. In a preferred aspect, the titration stage for patients who are not poor metabolisers is 5-7 days, for example 6 days.

In an aspect, where the patient is not a poor metabolizer patient, and the therapeutic dosage is 2.0 mg, the titration regimen may be day 1 - 0.25mg; day 2 - 0.25mg; day 3 - 0.5mg; day 4 - 0.75mg; day 5 - 1.25mg; day 6 - 2 mg (therapeutic dose).

In an aspect, the titration regimen of the present invention may be used to initiate treatment of patents at risk of cardiac side effects, for example patients at risk of heart failure, arrythmias, patients with high grade atrio-ventricular blocks or sick sinus syndrome, patients with a history of syncopal episodes, or patients requiring or under beta blockers, or patients requiring or under anti-arrhythmic treatment, such as patients under treatment with Class Ia (e.g. quinidine, procainamide) or Class III anti-arrhythmic drugs (e.g., amiodarone, sotalol).

The above titration regimen may also be used to reinitiate treatment on patients (poor metabolizer or non-poor metabolizer patients) who have undergone an interruption or treatment holiday in the maintenance dosage regime e.g. a holiday of greater than 3 days or 4 days, greater than 6, 8, 10, 12 or 14 days.

## Individualised dosing

In an embodiment, following the titration regimen or in cases where the titration regimen is not used, the effect of BAF312 on the patient lymphocyte count may be assessed following administration of BAF312 at the therapeutic dosage. In cases where the lymphocyte level is reduced below a level considered clinically optimal (for example to a level giving rise to an increased risk of opportunistic infection without increasing clinical benefit) following administration of BAF312 at the therapeutic dosage, the daily dosage may be reduced to a reduced dosage.

In this and related aspects (e.g the ninth and tenth aspects of the invention) the daily dosage reduction may take place over in a single step or in more than one step e.g. 2 or 3 steps. Preferably the dosage reduction takes place in a single step.

In an aspect, the dosage is reduced from a daily therapeutic dosage of 1.5-2.5mg, for example from 2mg.

In an aspect, the daily dosage is reduced to a dosage in the range from 0.5-1.5mg, for example 1.0mg.

In a preferred aspect, the daily dosage is reduced from 2mg to 1mg in a single step over subsequent days.

Following dosage reduction, the reduced dosage may be subsequently increased or maintained. In a preferred aspect, the reduced dosage is maintained.

The blood lymphocyte level considered below the clinically optimal level may be assessed by the physician. For example this lymphocyte level may be a level at which further reduction is determined to give rise to an increased risk of opportunistic infection without increasing clinical benefit. For example, the lymphocyte level may be less than or equal to 1.0x10e9/L, such as less than or equal to 0.8.0x10e9/L, less than or equal to 0.6x10e9/L, less than or equal to 0.4x10e9/L or less than or equal to 0.2x10e9/L.

In an aspect, the blood lymphocyte level considered below the clinically optimal level is a blood lymphocyte level less than or equal to 0.2x10e9/L.

Blood lymphoyte level may be measured using any standard technique such as e.g. flow cytometry.

In a preferred aspect of the invention, there is provided a method of optimizing the daily dosage of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof to patient in need, said method comprising the steps of:

(i) measuring the patient blood lymphocyte level following administration of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof at

a daily therapeutic dosage of about 2mg; and

(ii) in cases where the blood lymphocyte level is reduced below a level considered clinically optimal following daily administration of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof at the therapeutic dosage, reducing the daily dosage to about 1mg.

In this aspect, the lymphocyte level considered below the clinically optimal level may be less than or equal to 1.0x10e9/L, such as less than or equal to 0.8.0x10e9/L, less than or equal to 0.6x10e9/L, less than or equal to 0.4×10e9/L or less than or equal to 0.2x10e9/L.

In a preferred embodiment of this aspect, the lymphocyte level considered below the clinically optimal level may be less than or equal to 0.2x10e9/L.

Patient genotype testing

In vitro, CYP2C9 was identified as the major metabolizing enzyme in liver microsomes. Since this enzyme is genetically polymorphic, effects of genetic polymorphism to the oxidative metabolism was anticipated. In addition to classical enzyme phenotyping methods, the approach using individual microsomes from genotyped donors was designed to address this effect in vitro. Subsequent CYP2C9 genotype sensitivity analysis demonstrated substantial reduced metabolism activities in the liver microsomes of individual donors with special CYP2C9*2*2 and CYP2C9*3*3 genotypes as compared to the wild-type donors.

As is understood by the skilled person, the genotype of the patient plays an important role in determining the observed phenotype i.e. the observed capacity of the CYP2C9 enzyme to metabolise BAF312. In the case of the CYP2C9 enzyme, there is a close correlation between the measured CYP2C9 genotype and the observed poor metabolizer phenotype. Therefore the test of the genotype is a good predictor of the observed phenotype.

For the avoidance of doubt, in an aspect of the invention, the patient genotype (including whether or not the patient has the poor metabolizer genotype) may be tested by correlation with the observed phenotype.

The CYP2C9 phenotype may be tested by administering a probe substrate of CYP2C9 and calculation of the so called metabolic ratio (=plasma concentration of metabolite/parent compound).

Testing of the patient genotype for patients belonging to the poor metabolizer subclass may be carried out by any standard testing method e.g. by a standard genotyping method e.g. PCR screening. The patient genotype may be determined by an in vitro test method e.g. a genotyping method. For example, in vitro testing may be carried out by taking a body fluid (e.g. blood or saliva e.g. blood) or tissue sample from the patient and analyzing the sample by any standard testing method (e.g. PCR screening) to determine the patient genotype. In an embodiment, the patient genotype is determined by analysis of a blood, saliva or tissue sample taken from the patient. In a preferred embodiment, the patient genotype is determined by analysis of a blood sample taken from the patient.

The patient genotype may be tested in vivo by measuring the patient exposure to BAF312 following administration, and in cases where there is a good correlation between the observed metabolic behavior and a specific genotype, allocating the patient genotype on the basis of the observed phenotype behaviour.

Patient monitoring

In aspects of the invention where the level of patient monitoring by the medical practitioner following administration of BAF312 depends on the patient genotype e.g. the eleventh and other relevant aspects, the level of patient monitoring for patients without the poor metabolizer genotype may be either no monitoring or a low level of monitoring for a monitoring period e.g. remote monitoring without the patient visiting a medical facility e.g. remote monitoring using a heart monitor capable of measuring the patient's status and signaling in the case that an adverse event occurs.

Substantial patient monitoring includes continuous or periodic monitoring of the patient by a physician or other trained medical practitioner for adverse events e.g. in a medical facility such as a hospital or other treatment centre.

The monitoring period may be greater than or equal to 3hours, for example greater than or equal to 6 hours or greater than or equal to 12 hours or greater than or equal to one day. The monitoring period may also be less than one week, for example less than 4 days or less than 2 days. In an embodiment, the monitoring period is at least 6 hours.

Adverse events include heart rate reduction to <45 bpm e.g. <40bpm; new onset second degree atrioventricular block (AV block) or other events considered to require the attention of a medical practitioner until resolution.

Specific embodiments

The invention provides the following specific embodiments of the aspects of the invention:

A method of assessing the appropriate therapeutic dose of 1-{4-[1-(4-cyclohexyl-3-trifluoromethyl-benzyloxyimino)-ethyl]-2-ethyl-benzyl}-azetidine-3-carboxylic acid to administer to a patient in need thereof, comprising the steps of:

(i) testing whether or not the patient has the CYP2C9*3*3 genotype; and
(ii) if the patient does not have the CYP2C9*3*3 genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at a dose of about 2mg per day; or
(iii) if the patient does have the poor metaboliser genotype, either

(a) administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at a dose of about 0.25-0.75mg per day e.g. 0.5mg per day;
or
(b) not administering 1-{4-[1-(4-cyclohexyl-3-trifluoromethyl-benzyloxyimino)-ethyl]-2-ethyl-benzyl}-azetidine-3-carboxylic acid to the patient.

A method of initiating use of a drug recommended not to be used with 1-{4-[1-(4-cyclohexyl-3-trifluoromethyl-benzyloxyimino)-ethyl]-2-ethyl-benzyl}-azetidine-3-carboxylic acid, comprising the steps of:

(i) testing whether or not the patient has the CYP2C9*3*3 genotype; and
(ii) if the patient does not have the CYP2C9*3*3 genotype, inititiating use of the drug within 3-14 days after the last dose of 1-{4-[1-(4-cyclohexyl-3-trifluoromethyl-benzyloxyimino)-ethyl]-2-ethyl-benzyl}-azetidine-3-carboxylic acid has been administered to the patient; or
(iii) if the patient does have the poor metaboliser genotype genotype, initiating use of the drug within a of 21-49 days after the last dose of 1-{4-[1-(4-cyclohexyl-3-trifluoromethyl-benzyloxyimino)-ethyl]-2-ethyl-benzyl}-azetidine-3-carboxylic acid has been administered to the patient.

A method of treating a patient in need with 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, comprising the steps of:

(i) testing whether or not the patient has the CYP2C9*3*3 genotype; and
(ii) if the patient does not have the CYP2C9*3*3 genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at a dose of about 2mg per day; or
(iii) if the patient does have the CYP2C9*3*3 genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at a dose of about 2mg per day in combination with a CYP2C9 metabolic activity promoter.

A method for the treatment of an autoimmune condition in a patient in need thereof, said patient having the CYP2C9*3*3 genotype, comprising administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient in a daily amount in the range from 0.25 - 0.75mg e.g. in a daily amount of about 0.5mg.

1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, for use in a method of treating a patient suffering from an autoimmune condition, said patient having the CYP2C9*3*3 genotype, said method comprising administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient in a daily amount in the range from 0.25 - 0.75mg e.g. in a daily amount of about 0.5mg.

1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, for use in a method of treating a patient suffering from an autoimmune condition, said method comprising the steps of:

(i) testing whether or not the patient has the CYP2C9*3*3 genotype; and
(ii) if the patient does not have the CYP2C9*3*3 genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at a dose of about 2mg per day; or
(iii) if the patient does have the CYP2C9*3*3 genotype, either

(a) administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient in a daily amount in the range from

0.25-0.75mg e.g. ina daily amount of about 0.5mg;
or
(b) not administering 1-{4-[1-(4-cyclohexyl-3-trifluoromethyl-benzyloxyimino)-ethyl]-2-ethyl-benzyl}-azetidine-3-carboxylic acid to the patient.

1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, for use in a method of treating a patient in need, said method comprising the steps of:

(i) testing whether or not the patient has the CYP2C9*3*3 genotype; and
(ii) if the patient does not have the CYP2C9*3*3 genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at a dose of about 2mg per day; or
(iii) if the patient does have the CYP2C9*3*3 genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient patient at a dose of about 2mg per day in combination with a CYP2C9 metabolic activity promoter.

A method of optimizing the daily dosage of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof to patient in need, said method comprising the steps of:

(i) measuring the patient blood lymphocyte level following administration of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof at a dose of about 2mg per day; and
(ii) in cases where the blood lymphocyte level is reduced below a level of 0.2x10e9/L following daily administration of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof at the therapeutic dosage, reducing the the daily dosage to about 1mg per day.

1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, for use in a method of treating a patient in need, said method comprising the steps of:

(i) measuring the patient blood lymphocyte level following administration of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof at a dose of about 2mg per day; and
(ii) in cases where the blood lymphocyte level is reduced below 0.2x10e9/L following daily administration of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof at the therapeutic dosage, reducing the the daily dosage to about 1mg per day.

A method of treating a patient in need with 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, comprising the steps of:

(i) testing whether or not the patient has the CYP2C9*3*3 genotype; and
(ii) if the patient does not have the CYP2C9*3*3 genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at a dose of about 2mg per day with no or a low level of patient monitoring by a medical practitioner; or
(iii) if the patient does have the CYP2C9*3*3 genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at at a dose of about 2mg per day, with an increased level of patient monitoring by a medical practitioner.

1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, for use in a method of treating MS, said method comprising the steps of:

(i) testing whether or not the patient has the CYP2C9*3*3 genotype; and
(ii) if the patient does not have the CYP2C9*3*3 genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to

the patient at a dose of about 2mg per day with no or a low level of patient monitoring by a medical practitioner; or

(iii) if the patient does have the CYP2C9*3*3 genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at at a dose of about 2mg per day, with an increased level of patient monitoring by a medical practitioner.

1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, in the manufacture of a medicament for the treatment of an autoimmune disease, said treatment being by a method according to any of the preferred embodiments above.

In a preferred aspect for the above specific embodiments, the patient is a patient suffering from MS e.g. SPMS or rSPMS.

The invention is further illustrated by the following non-limiting examples.

## Example 1 - Identification of CYP2C9 as main metabolizing enzyme for 1-{4-[1-(4-cyclohexyl-3-trifluoromethyl-benzyloxyimino)-ethyl]-2-ethyl-benzyl]-azetidine-3-carboxylic acid

### List of abbreviations

*CHAPS* 3-[3-Cholamidopropyl)dimethylammonio]-1-propane-sulfonate

$CL_{int}$ Intrinsic clearance

*CYP* Cytochrome P450

*DDI* Drug-drug interaction

*DETC* diethyldithiocarbamate

*BT* Biotransformation

*HLM* Human liver microsomes

*HPLC* High performance liquid chromatography

$K_m$ Michaelis-Menten constant (substrate concentration producing half maximal velocity)

$K_m^{app}$ Apparent Michaelis-Menten constant

*LSC* Liquid scintillation counting

*min* minutes

$NADP^+$ Nicotinamide adenine dinucleotide phosphate-oxidized form

*NADPH* Dihydronicotinamide adenine dinucleotide phosphate (reduced form of $NADP^+$)

*R* Correlation coefficient between two data sets

*rhCYP* Recombinant human cytochrome P450

*rpm* Revolution per minute

*TAO* troleandomycin

*UV* Ultraviolet

$V_{max}$ Maximum velocity (reaction velocity at saturating substrate concentration)

### 1. Materials and methods

1.1 Test substance

| | |
|---|---|
| *Labeled compound:* | [14C]BAF312 The labeling and purification were carried out by the Isotope Laboratories Basel, Novartis Pharma AG, Switzerland |
| *Molecular formula / Weight (unlabeled)* | $C_{29}H_{35}F_3N_2O_3$/ 516.61 |
| *Radiochemical purity* | 98.9 % |
| *Specific activity* | 4.218 MBq/mg |

(continued)

*Chemical structure*

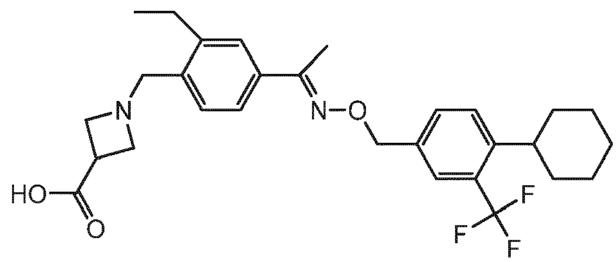

Stock solutions of 5 mM [14C]BAF312 were prepared in 100 mM phosphate buffer pH 7.4 containing 0.25% CHAPS (to increase solubility) and diluted appropriately for incubations in 100 mM phosphate buffer pH 7.4.

1.2 Chemicals

Furafylline, quercetin, quinidine, sulfaphenazole, ketoconazole, CHAPS, TAO and tranylcypromine were purchased from Sigma Chemicals (St. Louis, MO, USA). DETC was purchased from Fluka AG (Buchs, Switzerland) and triethylenethiophosphoramide from Acros Organics (Geel, Belgium)

Phosphate buffer pH 7.4 (100 mM) was prepared by mixing 60 mL of 100 mM KH2PO4 (Fluka AG, Buchs, Switzerland) solution and 470 mL 100 mM Na2HPO4 2H2O solution (Merck, Darmstadt, Germany). The pH was accurately adjusted to 7.4 with the 100 mM KH2PO4 solution. Distilled water (HPLC grade) was obtained from Fluka AG (Buchs, Switzerland). β-NADPH was from Sigma (St. Louis, MO, USA). Tris buffer pH 7.4 (1 M) was purchased from Applichem (Darmstadt, Germany) and further diluted to 0.1 M with water (Fluka).

Irga Safe Plus was used as liquid scintillation counting cocktail (ref. 6013249, Packard Bioscience, Meriden, CT, USA). For the on-line radiodetection in HPLC analysis, the liquid scintillator Rialuma® (Lumac-LSC, Groningen, The Netherlands) was used. Following chemicals were used to prepare the HPLC solvents: trifluoro acetic acid (analytical grade, ref 97100, Fluka), formic acid (analytical grade, ref 00264, Merck), acetonitrile (gradient grade, ref 0030, Merck) and water (chromatography grade, ref. 94486, Fluka). Other reagents, chemicals and buffer salts were from Merck (Darmstadt, Germany) or Fluka AG (Buchs, Switzerland) and were of analytical grade quality.

1.3 Human liver microsomes

A pool of liver microsomes prepared from 47 individual donors was obtained from BD Biosciences (Woburn, MA, USA, catalog N° 452161, Lot 26). Pathogenicity testing of each of the livers in the pool was performed using a PCR protocol. Each liver was found to be negative for HIV1&2, HTLV1&2, and hepatitis B&C. The total P450 content was 360 pmol/mg protein. Catalytic activities of enzymes were provided by the manufacturer (activities in pmol/(mg protein • min)): phenacetin O-deethylase (CYP1A2, 460), coumarin 7-hydroxylase (CYP2A6, 1300), (S)-mephenytoin N-demethylase (CYP2B6, 55), paclitaxel 6α-hydroxylase (CYP2C8, 190), diclofenac 4'-hydroxylase (CYP2C9, 2900), (S)-mephenytoin 4'-hydroxylase (CYP2C19, 56), bufuralol 1'-hydroxylase (CYP2D6, 94), chlorozoxazone 6-hydroxylase (CYP2E1, 2000), testosterone 6β-hydroxylase (CYP3A4, 4300), lauric acid 12-hydroxylase (CYP4A11, 1400), methyl p-Tolyl sulfide oxidase (FMO, 2100), estradiol 3-glucuronidation (UGT1A1, 1200), trifluoperazine glucuronidation (UGT1A4, 490), propofol glucuronidation (UGT1A9, 4900) and cytochrome c reductase (410).

1.4 Recombinant human P450 enzymes

Microsomes prepared from baculovirus infected insect cells (BTI-TN-5B1-4) expressing the following human P450 enzymes and the insect cell membrane preparations (negative control) were obtained from BD Biosciences (Woburn, MA, USA).

| Enzyme | Cat. No | Lot No | CYP Activity [a)] | P450 (pmol/mL) | Protein (mg/mL) |
|---|---|---|---|---|---|
| CYP1A1 + P450 reductase | 456211 | 15 | 27.8 | 1000 | 14.3 |
| CYP1A2 + P450 reductase | 456203 | 27 | 21 | 1000 | 7.0 |
| CYP1 B1 + P450 reductase | 456220 | 4 | 5 | 1000 | 4.7 |
| CYP2A6 + P450 reductase | 456204 | 14 | 9.6 | 2000 | 13 |
| CYP2B6 + P450 reductase | 456210 | 7 | 5 | 1000 | 8.3 |

(continued)

| Enzyme | Cat. No | Lot No | CYP Activity a) | P450 (pmol/mL) | Protein (mg/mL) |
|---|---|---|---|---|---|
| CYP2C8 + P450 reductase + $b_5$ | 456252 | 16 | 7.9 | 1000 | 2.6 |
| CYP2C9*1 + P450 reductase + $b_5$ | 456258 | 28 | 20 | 1000 | 2.4 |
| CYP2C18 + P450 reductase | 456222 | 12 | 0.8 | 1000 | 6.8 |
| CYP2C19 + P450 reductase | 456259 | 18 | 25 | 1000 | 4.3 |
| CYP2D6*1 + P450 reductase | 456217 | 15705 | 55 | 1000 | 6.6 |
| CYP2E1 + P450 reductase + $b_5$ | 456206 | 12 | 10 | 2000 | 6.3 |
| CYP2J2 + P450 reductase + $b_5$ | 456264 | 1 | 18 | 1000 | 4.8 |
| CYP3A4 + P450 reductase + $b_5$ | 456202 | 61 | 120 | 1000 | 6.9 |
| CYP3A5 + P450 reductase | 456235 | 16 | 4.4 | 2000 | 4.6 |
| CYP3A7 + P450 reductase + $b_5$ | 456237 | 7 | 1.1 | 1000 | 5.4 |
| CYP4A1 1 + P450 reductase | 456221 | 8 | 32 | 1000 | 8.7 |
| CYP4F2 + P450 reductase + $b_5$ | 456272 | 2 | 1.6 | 1000 | 1.8 |
| CYP4F3A + P450 reductase + $b_5$ | 456273 | 3 | 45 | 500 | 13 |
| CYP4F3B + P450 reductase + $b_5$ | 456274 | 3 | 12 | 1000 | 2.3 |
| CYP4F12 + P450 reductase + $b_5$ | 456275 | 1 | 9.9 | 1000 | 4.7 |
| CYP19 + P450 reductase | 456260 | 2 | 5.8 | 1000 | 4.2 |
| Insect control | 456201 | 52 | n.d. | n.d. | 5 |

a): pmol product /min/pmol protein (CYP1A1: 7-ethoxyresorufin deethylase, CYP1A2: phenacetin deethylase, CYP1B1: 7-ethoxyresorufin deethylase, CYP2A6: coumarin 7-hydroxylase, CYP2B6: 7-ethoxy-4-trifluoromethylcoumarin deethylase, CYP2C8: paclytaxel 6α-hydroxylase, CYP2C9: diclofenac 4'-hydroxylase, CYP2C18 (lot 1): 7-ethoxy-4-trifluoromethylcoumarin deethylase, CYP2C18 (lot 2 and 12): diclofenac 4'-hydroxylase, CYP2C19: (S)-mephenytoin 4'-hydroxylase, CYP2D6*1 and 2D6*10: bufuralol 1'-hydroxylase, CYP2E1: p-nitrophenol hydroxylase, CYP2J2: terfenadine hydroxylase, CYP3A4: testosterone 6β-hydroxylase, CYP3A5: testosterone 6β-hydroxylase, CYP3A7: testosterone 6β-hydroxylase, CYP4A11: lauric acid ω-hydroxylase, CYP4F2: 20-hydroxy leukotriene $B_4$, CYP 4F3A: 20-hydroxy leukotriene $B_4$, CYP 4F3B: 20-hydroxy leukotriene $B_4$, CYP4F12: terfenadine hydroxylase, CYP19: aromatase).
n.d.: not detectable

1.5 Incubation of [$^{14}$C]BAF312 with human liver microsomes and recombinant human CYPs

The incubations were carried out in 0.1 M phosphate buffer, pH 7.4. Typical incubations of 200 (or 400) μL total volume were prepared as follows: 10 μL of 100 mM MgCl2 (5 mM), stock solutions of CHAPS, substrate and microsomes or recombinant human cytochrome P450 isoenzymes were added to appropriate volume of the buffer. The detergent CHAPS was added to a final concentration of maximally 0.025% (w/v) into all incubation in order to increase the solubility of the test substance. Reaction was started by addition of 20 μL of a fresh 10 mM NADPH (1 mM). The final concentrations are given in parentheses. The final concentrations of organic solvent were maximally 0.5% (v/v). For some experiments, higher incubation volumes were prepared by keeping proportionally the quantity of all solutions. The samples were incubated at 37°C in a thermomixer (Eppendorf 5355) with agitation at 500 rpm.

The incubation reactions were stopped and the protein was precipitated by addition of an equal volume of ice-cold 0.5% formic acid in acetonitrile. After 30 min at -80°C (or overnight at -20°C) the samples were centrifuged at 30'000 × g for 15 min. The supernatant was withdrawn. Aliquots were analyzed by LSC (20 μL) and the supernatant was diluted appropriately with water to obtain a final solution containing less than 30% of acetonitrile. For samples with lower concentration in substrate, supernatants were evaporated to about the half of initial volumes under reduced pressure at 40°C with SpeedVac® concentrator (model AES 2010, Savant Inc., Holbrook, NY, USA) then mixed with 0.5% formic acid in acetonitrile to obtain a final solution containing less 30 % of acetonitrile. Sample solutions were analyzed by HPLC combined with radiodetection. (for HLPC method, see Section 1.6)

The residual pellet was rinsed twice with 0.5 mL mixture of 0.25% formic acid in water/acetonitrile (1:1, v/v) and dissolved (about one hour under shaking at 20°C) in 0.5 mL of a mixture containing 50% (v/v) Soluene-350 (0.5 M in toluene) and 50% isopropanol (v/v). Radiometry of aliquots of the supernatant and of the total amount of dissolved pellet was performed on a liquid scintillation counter (Tri-Carb 2500 TR, Packard Canberra Instr. Co. Meriden, CT, USA) after mixing with 10 mL liquid scintillation counting cocktail.

1.5.1 Human liver microsomes

Time dependence

| | |
|---|---|
| Human liver microsomes | 0.30 mg/mL |
| Incubation volume | 200 μL |
| Buffer | 100 mM phosphate, pH 7.4, 0.025% CHAPS, + 10 μL $MgCl_2$100 mM |
| Substrate (final concentration) | 1 and 10 μM |
| Solvent | No solvent |
| Pre-incubation | 3 min at 37°C |
| Reaction start | 20 μL β-NADPH 10 mM |
| Incubator | Eppendorf thermomixer, 500 rpm, 37°C |
| Incubation time | 0, 5, 10, 15, 20, 30, 45, 90, 120, 60, 90, 120, 150 and 180 min |
| Stopping agent | 200 μL ice-cold 0.5% formic acid in acetonitrile, 30 min at -80°C |
| Protein precipitation | Centrifugation for 15 min, 30,000 × g |
| Number of samples | 1 per condition |

Protein concentration dependence

| | |
|---|---|
| Human liver microsomes | 0, 0.025, 0.05, 0.01, 0.2, 0.3, 0.4, 0.5, 0.6, 0.8, 1 and 1.3 mg/mL |
| Incubation volume | 200 μL |
| Buffer | 100 mM phosphate, pH 7.4, 0.025% CHAPS + 10 μL MgCl2100 mM |
| Solvent | No solvent |
| Substrate (final concentration) | 10 μM |
| Pre-incubation | 3 min at 37°C |
| Reaction start | 20 μL β-NADPH 10 mM |
| Incubator | Eppendorf thermomixer, 500 rpm, 37°C |
| Incubation time | 60 min |
| Stopping agent | 200 μL ice-cold 0.5% formic acid in acetonitrile, 30 min at -80°C |
| Protein precipitation | Centrifugation for 15 min, 30,000 × g |
| Number of samples | 2 per condition |

Enzyme kinetics in HLM

| | |
|---|---|
| Human liver microsomes | 0.1 mg/mL |
| Incubation volume | 200 μL |
| Buffer | 100 mM phosphate, pH 7.4, 0.025% CHAPS + 10 μL MgCl2100 mM |
| Substrate (final concentration) | 1, 3, 5, 10, 15, 20, 30, 40, 60, 80, 100, 150, 200, 250, 300 μM concentrations |
| Solvent | No solvent |
| Pre-incubation | 3 min at 37°C |
| Reaction start | 20 μL β-NADPH 10 mM |
| Incubator | Eppendorf thermomixer, 500 rpm, 37°C |
| Incubation time | 90 min |
| Stopping agent | 200 μL ice-cold 0.5 % formic acid in acetonitrile, 30 min at -80°C |
| Protein precipitation | Centrifugation for 15 min, 30,000 × g |
| Number of samples | 2 per condition |

Inhibition by specific inhibitors

| | |
|---|---|
| Human liver microsomes | 0.1 mg/mL |
| Incubation volume | 200 μL |
| Buffer | 100 mM phosphate, pH 7.4, + 10 μL MgCl2100 mM |
| Substrate (final concentration) | 5 and 40 μM (in 0.0003% and 0.002% CHAPS) |
| Solvent | Methanol: ≤ 0.5 % (Stock solution in methanol): |
| Inhibitor (final concentration) | *furafylline (1A2 inhibitor): 2 and 10 μM<br>*diethyldithiocarbamate (DETC, 2E1 inhibitor): 5 and 30 μM<br>Quercetin (2C8 inhibitor): 2 and 10 μM<br>Triethylenethiophosphoramide (2B6 inhibitor): 5 and 20 μM<br>sulfaphenazole (2C9 inhibitor): 2 and 10 μM<br>tranylcypromine (2C19 inhibitor): 2 and 10 μM<br>quinidine (2D6 inhibitor): 0.1 and 1 μM<br>ketoconazole (3A4 inhibitor): 0.1 and 1 μM |
| Preincubation | 3 min at 37°C; *: 15 min at 37°C |
| Reaction start | 20 μL β-NADPH 10 mM *start reaction with substrate after 15 min preincubation at 37°C |
| Incubator | Eppendorf thermomixer, 500 rpm, 37°C |
| Incubation time | 90 min |
| Stopping agent | 200 μL ice-cold 0.5 % formic acid in acetonitrile, 30 min at -80°C |
| Protein precipitation | Centrifugation for 15 min, 30,000 g |
| Number of samples | 2 per condition |

HLM from single donors with different CPY2C9 genotypes

| | |
|---|---|
| Human liver microsomes | 0.1 mg/mL<br>HLM single donor with CYP2C9 genotype 1*/1* (HG6), 2*/2* (HG103), 3*/3* (HK27) |
| Incubation volume | 200 μl |
| Buffer | 100 mM Tris (pH 7.4) containing 0.0005% CHAPS + 10 μL MgCl2100 mM |
| Substrate (final concentration) | 5 μM [14C]BAF312 |
| Solvent | No solvent |
| Preincubation | 3 min at 37°C |
| Reaction start | 20 μL β-NADPH 10 mM |
| Incubator | Eppendorf thermomixer, 500 rpm, 37°C |
| Incubation time | 60 min |
| Stopping agent | 200 μL ice-cold 0.5 % formic acid in acetonitrile, 30 min at -80°C |
| Protein precipitation | Centrifugation for 15 min, 30,000 × g |
| Number of samples | 2 per condition |

1.5.2 Recombinant CYPs

Enzyme mapping

| | |
|---|---|
| Isoenzymes | 30 pmol CYP/mL<br>insect cell control, CYP1A1, CYP1A2, CYP1B1, CYP2A6, CYP2B6, CYP2C8, CYP2C9, CYP2C18, CYP2C19, CYP2D6*1, CYP2E1, CYP2J2, CYP3A4, CYP3A5, CYP3A7, CYP4A11, CYP4F2, CYP4F3A, CYP4F3B, CYP4F12, CYP19<br>HLM (pool, 0.3 mg/mL), |
| Incubation volume | 200 μL |
| Buffer | 100 mM phosphate, pH 7.4, 0.025% CHAPS + 10 μL MgCl2100 mM |
| Substrate (final concentration) | 10 and 40 μM |
| Solvent | No solvent |
| Pre-incubation | 3 min at 37°C |
| Reaction start | 20 μL β-NADPH 10 mM |
| Incubator | Eppendorf thermomixer, 500 rpm, 37°C |
| Incubation time | 30 min |
| Stopping agent | 200 μL ice-cold 0.5 % formic acid in acetonitrile, 30 min at -80°C |
| Protein precipitation | Centrifugation for 15 min, 30,000 × g |
| Number of samples | 2 per condition |

Enzyme kinetics CYP3A4

| | |
|---|---|
| Recombinant CYPs | 3A4, Lot 61 |
| Enzyme concentration | 50 pmol/mL (0.345 mg protein/mL) |
| Incubation volume | 200 μL |
| Buffer | 100 mM phosphate, pH 7.4, 0.025% CHAPS + 10 μL MgCl2100 mM |
| Solvent | No solvent |
| Substrate (final concentration) | 5, 10, 15, 20, 30, 40, 60, 80, 100, 150, 200, 250, 300 μM |
| Pre-incubation | 3 min at 37°C |
| Reaction start | 20 μL β-NADPH 10 mM |
| Incubator | Eppendorf thermomixer, 500 rpm, 37°C |
| Incubation time | 30 min |
| Stopping agent | 200 μL ice-cold 0.5 % formic acid in acetonitrile, 30 min at -80°C |
| Protein precipitation | Centrifugation for 15 min, 30,000 x g |
| Number of samples | 2 per condition |

Enzyme kinetics CYP2C9

| | |
|---|---|
| Recombinant CYPs | 2C9, Lot 28 |
| Enzyme concentration | 50 pmol/mL (0.12 protein mg/mL) |
| Incubation volume | 200 μL or more |
| Buffer | 100 mM phosphate, pH 7.4, 0.025% CHAPS + 10 μl MgCl2100 mM |
| Solvent | No solvent |
| Substrate (final concentration) | 5, 10, 15, 20, 30, 40, 60, 80, 100, 150, 200, 250, 300 μM |
| Pre-incubation | 3 min at 37°C |
| Reaction start | 20 μL β-NADPH 10 mM |
| Incubator | Eppendorf thermomixer, 500 rpm, 37°C |
| Incubation time | 60 min |
| Stopping agent | 200 μL ice-cold 0.5 % formic acid in acetonitrile, 30 min at -80°C |
| Protein precipitation | Centrifugation for 15 min, 30,000 x g |

(continued)

| | |
|---|---|
| Number of samples | 2 per condition |

1.6 HPLC instrumentation and conditions

| | |
|---|---|
| Equipment: | Agilent 1100 HPLC system |
| Solvent delivery system | A binary pump (model G1312A, Agilent Technologies, Waldbronn, Germany) |
| Autosampler | Model G2260A, Agilent Technologies |
| Sample Injection | via 900 μL stainless steel sample loop |
| UV detector | Model G1365B, Agilent Technologies |
| | Fixed wavelength 265 nm with a standard flow cell (model G1315-60012, 13 μL volume, 10 mm path length, 120 bar). |
| Software | Agilent ChemStation for LC 3D, Revision A.09.01 Radioactivity monitoring: Radiostar, (Berthold, Wildbad, Germany), Version 3.0 |
| Radioactivity monitoring | On-line radioactivity was monitored using a HPLC radioactivity detector (model LB 506 C-1, Berthold Technologies GmbH, Regensdorf, Switzerland), with a 0.5 mL liquid scintillator cell Z 500-4. The HPLC effluent were mixed with Rialuma®(Lumac, Groningen, Netherlands), pumped at a flow rate of 3 mL/min (Berthold pump model LB 5035) |

Chromatography conditions:

| | |
|---|---|
| Column | Nucleosil 100 C18 Nautilus, 250 x 4 mm I.D., 5 μm (Macherey-Nagel, order No 721130.40, Düren, Germany) |
| Pre-column | Nucleosil 100 C18 Nautilus, 8 x 4 mm I.D., 5 μm (order No 721140.40) |
| Column temperature | 40°C (Agilent column oven G1316A) |
| Flow rate | 1 mL/min (total solvent flow A + B) |
| Solvents | A: 0.5% formic acid + 0.1% trifluoroacetic acid in water |
| | B: 0.5% formic acid + 0.1% trifluoroacetic acid in acetonitrile |

HPLC gradient:

| HPLC Gradient (linear ramps) | |
|---|---|
| Time (min) | % B |
| 0 | 40 |
| 3 | 40 |
| 5 | 45 |
| 20 | 55 |
| 22 | 100 |
| 30 | 100 |
| 30.5 | 40 |
| 36 | 40 |

1.7 Analysis of enzyme kinetics

Enzyme kinetic parameters Vmax and Km of the biotransformation by HLM and major metabolizing enzymes were calculated by using SigmaPlot Version 8.0 (S1), Enzyme Kinetics module Version 1.1 software (SPSS Science Inc., Chicago, IL, USA). The intrinsic clearance was calculated by the equation: CLint = Vmax/Km.

Mean specific CYP enzyme concentrations in human liver microsomes were obtained from literature values (Rowland Yeo K, Rostami-Hodjegan A and Trucker GT (2004)] Abundance of cytochromes P450 in human liver: a meta-analysis. Br. J. Clinical Pharmacology; 57:687-688). For some recombinant human CYPs, the enzyme kinetic parameters were estimated by calculation assuming Michaelis-Menten type behavior for the two concentrations used, solving a linear equation system for 2 linear equations with 2 variables:

$$V_1 = \frac{Vmax \bullet [S_1]}{Km + [S_1]}, \quad V_2 = \frac{Vmax \bullet [S_2]}{Km + [S_2]}$$

## 2. Results

2.1 Evaluation of the incubation conditions

In an initial set of experiments, the linear range of the in vitro biotransformation of BAF312 with respect to incubation time was assessed. Incubations of 1 and 10 μM [$^{14}$C]BAF312 with 0.3 mg/mL protein concentration of human liver microsomes were performed from 0 to 180 min and the disappearance of parent compound in the supernatant was determined by HPLC with radiodetection. As shown in Figures 1-1, total metabolism of BAF312 in human liver microsomes increased in a linear manner up to 90 min incubation time (10 μM substrate) and 180 min incubation time (1 μM substrate). A second series of incubations at fixed incubation time (60 min) using 10 μM [$^{14}$C]BAF312 and different microsomal protein concentrations (0-1.3 mg/mL) resulted in a linear enzyme concentration dependent increase of biotransformation up to about 0.1 mg/ml (Figure 1-2). Consequently, the biotransformation of [$^{14}$C]BAF312 was investigated in incubations performed under linear conditions with respect to time (90 min) and protein content (0.1 mg/ml).

2.2 Concentration dependent biotransformation of [$^{14}$C]BAF312 in human liver microsomes

After establishing linear reaction conditions, enzyme kinetics parameters Km and Vmax were determined by incubating pooled human liver microsomes (0.1 mg/mL) with 15 substrate concentrations ranging from 1 to 300 μM for 90 min. (Table 1-1). Experimental data (rates of total metabolites formation) were analyzed by nonlinear regression analysis considering different kinetic models (Michaelis-Menten, Hill, isoenzyme, random substrate activation, substrate inhibition) as provided by the Enzyme Kinetics module, SigmaPlot (S1). Plotting the experimental data as Michaelis-Menten and Eadie-Hofstee plot (V vs. V/S) (see Figure 1-3), the kinetic data were fitted with substrate inhibition (uncompetitive) kinetic model. From the equation of this model, the apparent kinetic constants of total metabolism Km of 50.3 ± 9.7 μM and Vmax of 191 ± 25 pmol/min/mg were calculated. The derived intrinsic clearance (Vmax/Km) of the hepatic metabolism of BAF312 was 3.8 μL/mg/min.

2.3 Biotransformation of [$^{14}$C]BAF312 by recombinant human CYP isoenzymes

Microsomes prepared from baculovirus infected insect cells (BTI-TN-5B1-4) expressing one single human cytochrome P450 isoenzyme were used to assess the involvement of specific enzymes in the biotransformation of [14C]BAF312. Incubation experiments with a panel of 21 recombinant human CYPs (CYP1A1, 1A2, 1B1, 2A6, 2B6, 2C8, 2C9*1, 2C18, 2C19, 2D6*1, 2E1, 2J2, 3A4, 3A5, 3A7, 4A11, 4F2, 4F3A, 4F3B, 4F12 and CYP19) were conducted under similar conditions for each isoenzyme with 10 and 40 μM BAF312 incubation and 30 pmol CYP/mL for 30 min. At both concentrations (Table 1-2, Figure 1-4) CYP2C9*1 showed significant turnover under the experimental conditions used. Lower metabolism activities were also observed in incubation with CYP3A4, while some trace metabolism was detected with other CYP isoenzymes (2B6, 2C8, 2C19, 2J2, 3A5, 1A1).

CYP2C9 was found to be the most efficient P450 isoenzyme for BAF312 metabolism.

CYP2C9 is subject to significant genetic polymorphism (CYP2C9*1, CYP2C9*2, CYP2C9*3) which vary largely between different ethnic populations. Sulfaphenazole is a potent inhibitor of CYP2C9, both in vitro and in vivo.

Recombinant CYP3A4 and CYP3A5 were capable to metabolize BAF312 with low activity.

Enzyme kinetics parameters of the isoenzymes CYP2C9 and CYP3A4 for BAF312 metabolism were determined by incubating different concentrations of [$^{14}$C]BAF312 with the isoenzyme. The kinetic profile with CYP2C9 and 3A4 are

shown in Figure 1-5 and Figure 1-6 . Kinetic constants for both CYP3A4 (Km: 85.1 ± 8.6 μM; Vmax: 706 ± 31 pmol/min/nmol) and CYP2C9 (Km: 34.5 ± 5 μM; Vmax: 2596 ± 233 pmol/min/nmol) were determined. The derived intrinsic clearances (Vmax/Km) of the total metabolite formation of BAF312 are 8.3 μL/nmol/min and 75.2 μL/nmol/min for CYP3A4 and 2C9, respectively.

For other BAF312 metabolizing CYPs, kinetic constants Km and Vmax were estimated by solving 2 linear equations with 2 variables (Section 1.7), which allowed to estimate the enzymatic efficiency, or "intrinsic clearance" CLint for the various enzymes.

With regard to their importance in hepatic metabolic clearance of BAF312, an intrinsic clearance CLint relative to their abundance in human liver microsomes (Rowland Yeo, et al 2004) were calculated (Table 1-3). CYP2C9 contributed predominantly (79.2%) to the total intrinsic clearance in human liver microsomes. CYP3A (including 3A4 and 3A5) contribute for 18.5%. The contributions of other known drug-metabolizing enzymes (CYP2B6, 2C8, 2C19) were low.

2.4 Inhibition of [$^{14}$C]BAF312 biotransformation by chemical inhibitors

In vitro experiments using selective chemical inhibitors (Newton, et al, 1995; Tucker, et al 2001) to attenuate the microsomal metabolism of BAF312 by specific CYP enzymes in human liver microsomes (Table 1-4) were carried out. The biotransformation of BAF312 was tested at 5 μM substrate concentration in the presence of the 8 individual chemical inhibitors. With 1 μM ketoconazole, the metabolism rates of BAF312 were inhibited by 25%. Significant strong inhibition was observed with 2 μM and 10 μM sulfaphenazole (65-77%), which is a specific inhibitor for CYP2C9. Quercetine exhibited slight inhibition (11-31%). Tranylcypromine showed also a slight inhibition(11-29 %). Other chemical inhibitors tested did not substantially inhibit BAF312 metabolism (Table 1-4).

2.5 In vitro sensitivity analysis of different CYP2C9 genotype

CYP2C9 is a polymorphic isoenzyme with variable metabolic capacity in different subjects. Since the biotransformation of BAF312 to its hydroxylated metabolites was mainly catalyzed by this isoenzyme, significant effect of genetic polymorphism of this isoenzyme to the metabolism can be anticipated. To further investigate this issue sensitivity analysis was carried out in vitro. The metabolism of [$^{14}$C]BAF312 was studied in liver microsomes from individual donors with special genotypes (Table 1-5) for this enzyme. Figure 1-8 showed the comparison of biotransformation rates from CYP2C9*1/*1, 2C9*2/*2 and 2C9*3/*3 donors. Comparing to CYP2C9*1/*1 (wild-type), the metabolism rates of BAF312 in HLM from CYP2C9*2/*2 and 2C9*3/*3 donors were substantially lower. In human liver microsomes, a significant 10-fold decrease in the rate of hydroxylated metabolites formation was evident in CYP2C9*3/*3 samples compared to CYP2C9*1/*1 samples. There was also a marked reduction (up to 65%) of hydroxylated metabolites formation in the liver sample genotyped as CYP2C9*2/*2. These results confirms the potential of genetic polymorphism in the oxidative metabolism of this compound.

CYP enzyme phenotyping are traditionally performed by three basic approaches (specific chemical inhibitors or inhibitory antibodies, recombinant cytochrome P450s and correlation analysis) documented in the scientific literature and recognized by FDA (Bjornsson et al., 2003; Ogilvie et al., 2008). Each of these approach has its advantages but also shortcomings and so, a combination of approaches is highly recommended (at least two should be used, provided the results of both methods are similar). For BAF312, the in vitro biotransformation in liver microsomes from individual donors with special CYP2C9 genotypes was investigated. Significant effect of genetic polymorphism of CYP2C9 was demonstrated. Results of this additional methods were in line with the data from two approaches (chemical inhibition and recombinant enzyme kinetics) as decribed and therefore provided further support and additional confidence to the conclusion of BAF312 enzyme phenotyping. Application of genotype sensitivity analysis using individual microsomes from genotyped donors provides an additional approach as an alternative method for the reaction phenotyping of xenobiotics, particularly when their metabolism is catalyzed predominantly by a genetic polymorphic enzyme.

Collectively, all the in vitro data obtained from three independent phenotyping approaches conclude that CYP2C9 contributes predominantly to the human liver microsomal biotransformation of [$^{14}$C]BAF312 with partial contribution from CYP3A. Other CYP enzymes may also contribute to a minor extent.

**References to published literature**

Bjornsson TD, Callaghan JT, Einolf HJ, Fischer V, Gan L, Grimm S, Kao J, King SP, Miwa G, Ni L, Kumar G, McLeod J, Obach RS, Roberts S, Roe A, Shah A, Snikeris F, Sullivan JT, Tweedie D, Vega JM, Walsh J, and Wrighton SA (2003) The conduct of in vitro and in vivo drug-drug interaction studies: A Pharmaceutical Research and Manufacturers of America (PhRMA) perspective. Drug Metab Dispos 31:815-832.

[Guengerich FP (1996)] In vitro techniques for studying drug metabolism. Journal of Pharmacokinetics & Biopharmaceutics, 24:521-533.

[Newton DJ, Wang R W, Lu AYH (1995)] Cytochrome P450 inhibitors: Evaluation of specificities in the in vitro metabolism of therapeutic agents by human liver microsomes. Drug Metabolism and disposition; 25:154-158.

Ogilvie BW, Usuki E, Yerino P, and Parkinson A (2008) In vitro approaches for studying the inhibition of drug-metabolizing enzymes and identifying the drug-metabolizing enzymes responsible for the metabolism of drugs (Reaction phenotyping ) with emphasis on cytochrome P450, in: Drug-drug Interactions (Rodrigues AD ed), pp 231-358, Informa Healthcare, New York.

[Rettie AE and Jones JP GT (2005)] Clinical and toxicological relevance of CYP2C9: drug-drug interactions and pharmacogenetics. Annu. Rev. Pharmacol; 45:477-494.

[Rowland Yeo K, Rostami-Hodjegan A and Trucker GT (2004)] Abundance of cytochromes P450 in human liver: a meta-analysis. Br. J. Clinical Pharmacology; 57:687-688.

[Shimada T, Yamazaki H, Mimura M, et al (1994)] Interindividual variations in human liver cytochrome P-450 enzymes involved in the oxidation of drugs, carcinogens and toxic chemicals: studies with liver microsomes of 30 Japanese and 30 Caucasians. JPET; 270: 414-423.

[Tucker GT, Houston JB, Huang SM (2001)] Optimizing drug development: strategies to assess drug metabolism/transporter interaction potential-towards a consensus. Br J Clin Pharmacol; 52:107-17.

**References to computer software**

S1 Enzyme kinetics Module Version 1.1 for SigmaPlot 2002, Version 8.0, SPSS Science Inc., Chicago, IL, USA

S2 Simcyp Version 4.10.02, Simcyp Ltd., Sheffield, UK.

**Tables**

*Table 1.1 Concentration-dependent biotransformation of [$^{14}$C]BAF312*

The concentration-dependent biotransformation of [$^{14}$C]BAF312 by HLM (0.1 mg protein/mL) was investigated after 90 min incubations. The formation of metabolites (M5, M6, M7) in the supernatant was determined by HPLC analysis combined with radiodetection. Mean values of 2 incubations were given.

| mean [$^{14}$C]BAF312 Concentration [μM] | velocity of Total metabolites formation [pmol/min/mg protein] |
|---|---|
| 0.97 | 4.2 |
| 2.88 | 11.3 |
| 4.81 | 18.6 |
| 9.64 | 31.9 |
| 14.49 | 42.0 |
| 19.36 | 46.0 |
| 29.04 | 60.2 |
| 38.73 | 66.9 |
| 58.29 | 68.7 |
| 77.67 | 73.3 |
| 97.04 | 67.2 |
| 145.58 | 57.5 |
| 194.06 | 51.1 |

(continued)

| The concentration-dependent biotransformation of [$^{14}$C]BAF312 by HLM (0.1 mg protein/mL) was investigated after 90 min incubations. The formation of metabolites (M5, M6, M7) in the supernatant was determined by HPLC analysis combined with radiodetection. Mean values of 2 incubations were given. | |
|---|---|
| mean [$^{14}$C]BAF312 Concentration [μM] | velocity of Total metabolites formation [pmol/min/mg protein] |
| 242.39 | 37.5 |
| 290.94 | 35.0 |

*Table 1.2 Metabolite formation by recombinant P450 isoenzymes*

The biotransformation of [$^{14}$C]BAF312 (10 μM and 40 μM) by recombinant microsomes expressing a single human cytochrome P450 isoenzyme (30 pmol/mL) and by HLM (108 pmol/mL) was investigated after 30 minutes of incubation. The formation of metabolites was determined by HPLC analysis combined with radiodetection.

| Enzyme | Gentest Cat. No. | Lot. Nr. | Metabolism Rate (pmol/min/nmol CYP 450) | |
|---|---|---|---|---|
| | | | 10 μM BAF312 | 40 μM BAF312 |
| HLM pool | 456201 | 26 | 5.9 | 25.3 |
| Insect control | 452161 | 52 | nd | nd |
| CYP 1A1 | 456211 | 15 | 13.3 | 28.9 |
| CYP 1A2 | 456203 | 27 | nd | nd |
| CYP 1B1 | 456220 | 4 | nd | nd |
| CYP 2A6 | 456204 | 14 | nd | nd |
| CYP 2B6 | 456210 | 7 | 17.8 | 44.4 |
| CYP 2C8 | 456252 | 16 | 11.1 | 13.3 |
| CYP 2C9 | 456258 | 28 | 129.4 | 211.1 |
| CYP 2C18 | 456222 | 12 | nd | nd |
| CYP 2C19 | 456259 | 18 | 6.7 | 22.2 |
| CYP 2D6*1 | 456217 | 15705 | nd | nd |
| CYP 2E1 | 456206 | 12 | nd | nd |
| CYP 2J2 | 456264 | 1 | 2.8 | 8.9 |
| CYP 3A4 | 456202 | 61 | 23.3 | 53.3 |
| CYP 3A5 | 456235 | 16 | 3.9 | 2.2 |
| CYP 3A7 | 456237 | 7 | nd | nd |
| CYP 4A11 | 456221 | 8 | nd | nd |
| CYP 4F2 | 456272 | 2 | nd | nd |
| CYP 4F3A | 456273 | 3 | nd | nd |
| CYP 4F3B | 456274 | 3 | nd | nd |
| CYP 4F12 | 456275 | 1 | nd | nd |
| CYP 19 | 456260 | 2 | nd | nd |

**nd: not detectable**

*Table 1-3 Enzyme kinetic constants and estimated contribution of various CYPs to the metabolic clearance of BAF312 in human liver microsomes*

| Enzyme | **$K_m$** (μM) | **$V_{max}$** (pmol/ (min·nmol CYP) | **CL**($V_{max}$/$K_m$) μL/(min.·nmol CYP) | abundance[A] (pmol P450/mg) | Specific Cone. (% total CYP) | Rel. CL μl/ (min.nmol total CYP) | **Contribution** (% total Rel. CL) |
|---|---|---|---|---|---|---|---|
| CYP 1A1 | 25.5 | 47.3 | 1.9 | | | | |

(continued)

| Enzyme | **$K_m$** (μM) | **$V_{max}$** (pmol/ (min·nmol CYP) | **CL**($V_{max}/K_m$) μL/(min.·nmol CYP) | abundance[A] (pmol P450/mg) | Specific Cone. (% total CYP) | Rel. CL μl/ (min.nmol total CYP) | **Contribution** (% total Rel. CL) |
|---|---|---|---|---|---|---|---|
| CYP 1A2 | | | | 52 | 9.47% | | |
| CYP 1B1 | | | | | | | |
| CYP 2A6 | | | | 36 | 6.56% | | |
| CYP 2B6 | 40.0 | 88.9 | 2.2 | 11 | 2.00% | 0.04 | 0.4% |
| CYP 2C8 | 2.9 | 14.3 | 5 | 24 | 4.37% | 0.22 | 1.7% |
| CYP 2C9 | 34.5 | 2596 | 75.2 | 73 | 13.30% | 10.01 | 79.2% |
| CYP 2C18 | | | | | 0.20% | | |
| CYP 2C19 | 140 | 100 | 0.7 | 14 | 2.55% | 0.02 | 0.1% |
| CYP 2D6 | | | | 8 | 1.46% | | |
| CYP 2E1 | | | | 61 | 11.11% | | |
| CYP 2J2 | 374 | 106.7 | 0.3 | | | | |
| CYP 3A4[B] | 85.1 | 706 | 8.3 | 155 | 28.23% | 2.34 | 18.5% |
| CYP 3A5 | | | | | | | |
| CYP 3A7 | | | | | | | |
| CYP 4A11 | | | | | | | |
| CYP 4F2 | | | | | | | |
| CYP 4F3A | | | | | | | |
| CYP 4F3B | | | | | | | |
| CYP 4F12 | | | | | | | |
| Total | | 434 | 79% | 12.63 | | 100% | |

Footnote to Table 1-3: Enzyme kinetic parameters $V_{max}$ and $K_m$ of CYP3A4 and CYP2C9 were obtained from kinetic experiments. The values of other isoenzymes were estimated by solving the Michaelis-Menten equation V=Vmax·S/(Km+S) for the 2 concentrations of substrate and their rate numbers. A: the CYP abundance values were from Rowland Yeo et al (2004) and Simcyp software; B: for CYP3A4, the abundance value of CYP3A was used.

*Table 1-4 Inhibition of metabolism by CYP450 isoenzymes specific inhibitors in human liver microsomes*

The biotransformation of [$^{14}$C]BAF312 in human liver microsomes (0.1 mg/mL) was investigated in the absence and presence of specific inhibitor. The formation of metabolites was determined by HPLC analysis combined with radiodetection.

| Inhibitor | 5 μM BAF312 | |
|---|---|---|
| | Rate (pmol/min/mg) | Relative activity (%) |
| No Inhibitor (A) | 17.3 | 100 |
| No Inhibitor (B) | 17.7 | 100 |
| 2 μM Furafylline (CYP1A2) | 16.7 | 95 |
| 10 μM Furafylline (CYP1A2) | 16.1 | 91 |
| 5 μM Triethylenethiophosphoramide (CYP2B6) | 18.3 | 106 |
| 20 μM Triethylenethiophosphoramide (CYP2B6) | 18.8 | 109 |
| 2 μM Quercetin (CYP2C8) | 15.3 | 89 |
| 10 μM Quercetin (CYP2C8) | 11.9 | 69 |
| 2 μM Sulfaphenazole (CYP2C9) | 6.1 | 35 |
| 10 μM Sulfaphenazole (CYP2C9) | 3.9 | 23 |
| 2 μM Tranylcypromine (CYP2C19) | 15.3 | 89 |
| 10 μM Tranylcypromine (CYP2C19) | 12.2 | 71 |
| 0.1 μM Quinidine (CYP2D6) | 15.6 | 90 |
| 1 μM Quinidine (CYP2D6) | 16.4 | 95 |
| 5 μM DETC (CYP2E1) | 15.8 | 89 |
| 30 μM DETC (CYP2E1) | 13.6 | 77 |
| 0.1 μM Ketoconazole (CYP3A4) | 16.2 | 94 |
| 1 μM Ketoconazole (CYP3A4) | 13.0 | 75 |

A: normal blank; B: Blank with 15 min pre-incubation

*Table 1-5 CYP2C9 genotypes comparison of [$^{14}$C]BAF312 metabolism rates from HLM of individual donors*

| **Individual donors (obtained from BD Bioscience)** | | | | **Catalytic activity (provided by the manufacturer)** | | **Rate of metabolite formation** |
|---|---|---|---|---|---|---|
| **CYP2C9 genotype** | **Order No.** | **Lot No.** | **Designation** | **CYP2C9 (diclofenac 4'-hydroxylation) pmol/min/mg protein** | **CYP 3A4 (testosterone 6β-hydroxylation) pmol/min/mg protein** | **pmol/min/ mg protein** |
| **CYP2C9*1/*1** | **452006** | **2** | **HG 6** | **2700** | **3000** | **30.4** |
| **CYP2C9*2/*2** | **452103** | **1** | **HG 103** | **2200** | **2800** | **10.5** |
| **CYP2C9*3/*3** | **452027** | **1** | **HK27** | **480** | **4900** | **2.7** |

**Example 2 - In vivo effect of CYP2C9*3*3 gentoype on metabolisation of 1-{4-[1-(4-cyclohexyl-3-trifluoromethyl-benzyloxyimino)-ethyl]-2-ethyl-benzyl]-azetidine-3-carboxylic acid**

As part of a larger trial, two CYP2C9*3*3 genotyped patients (poor metabolizer) were administered 1-{4-[1-(4-cyclohexyl-3-trifluoromethyl-benzyloxyimino)-ethyl]-2-ethyl-benzyl}-azetidine-3-carboxylic acid and the resultant drug levels in the patient blood plasma samples measured and compared to those of CYP2C9*1*1 (normal metabolizer) patients.

The two CYP2C9*3*3 patients were found to experience an approximately 4-fold higher drug exposure than those of the CYP2C9*1*1 patients. This preliminary in vivo data confirmed previous Simcyp simulations, which predicted 4.1-fold mean AUC ratio in the groups of 45 virtual Simcyp populations based on the in vitro results as described in

Example 1 (section 2.5).

A further investigation could be carried out for example as described in the study plan below.

Study plan

A multi-center, open-label study in healthy volunteers with different CYP2C9 genotypes may be used to evaluate the effect of CYP2C9 sub-type on metabolism of 1-{4-[1-(4-cyclohexyl-3-trifluoromethyl-benzyloxyimino)-ethyl]-2-ethyl-benzyl}-azetidine-3-carboxylic acid.

The study may be divided into two parts: Part 1 and Part 2 .

All subjects will complete Part 1, undergo a wash-out period of six weeks (42 days) before the end of study visit for subjects carrying CYP2C9 *1/*1 genotype (EM) whereas subjects with *2/*3 and *3/*3 genotypes (PM) are expected to continue in Part 2. Replacement subjects for Part 2 do not need to undergo Part 1 prior to entering the study.

During Part 1, a total of up to 36 subjects will receive study drug:

- 6 to 18 CYP2C9 *1/*1 subjects,
- 6 to 9 CYP2C9 *2/*3 subjects,
- 6 to 9 CYP2C9 *3/*3 subjects.

The CYP2C9 *1*1 subjects will be matched by body weight (+/- 10%) to CYP2C9*2/*3 and *3/*3 subjects. During Part 2 a total of up to 18 subjects with CYP2C9 PM phenotype will receive study drug:

- 6 to 9 CYP2C9 *2/*3 subjects,
- 6 to 9 CYP2C9 *3/*3 subjects.

Study design

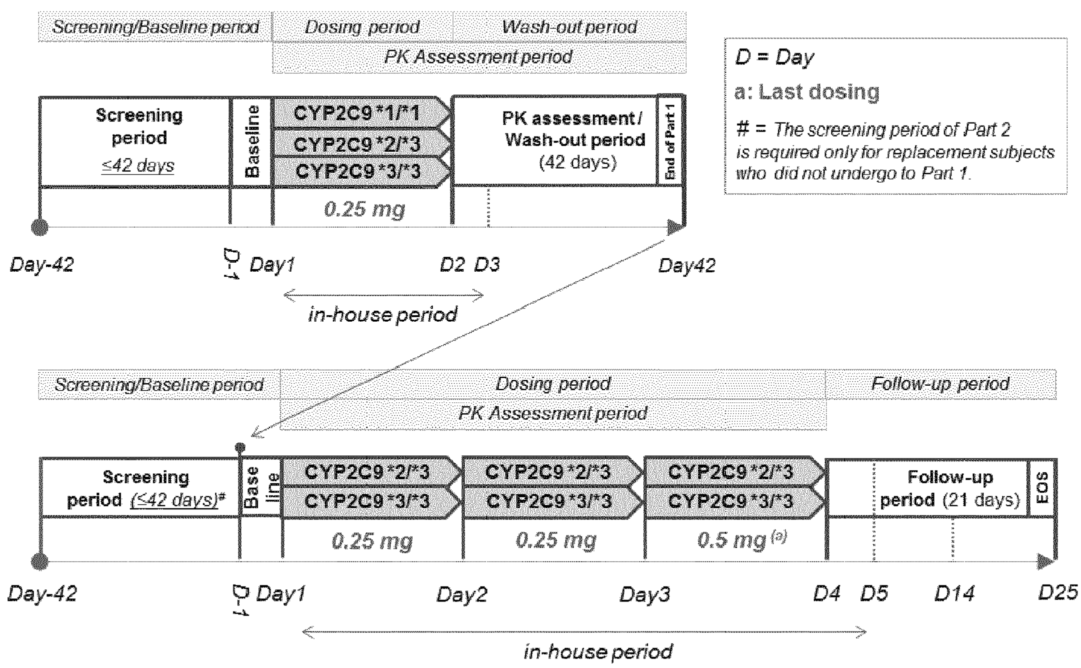


**Part 1:**

This study Part 1 will consist of a up to 41 day screening period, a baseline day (Day -1), a treatment day (Day 1), followed by a 6 week wash-out, PK assessment period up to Day 42 (corresponding to approx.6 times the predicted T1/2 of 170h in CYP2C9*3*3 carriers). PK assessments will be performed at the time-points shown in the Assessment Schedule.

Subjects who meet the eligibility criteria at screening will be admitted to baseline evaluations. All baseline safety

evaluation results must be available prior to dosing. Subjects will be admitted to the study site in the morning of the day prior to dosing (Day - 1) for baseline evaluations. The first day of dosing will occur on Day 1 when a single dose of 0.25 mg siponimod will be administered.

The completion evaluations for Part 1 will take place after a complete wash-out period (i.e. 6 weeks from dosing).

Safety assessments will include physical examinations, vital signs, standard clinical laboratory evaluations (hematology including lymphocyte count, blood chemistry, and urinalysis), 12-Lead ECG, on-line cardiac monitoring, Holter-ECG recording, adverse event, and serious adverse event monitoring.

Subjects will be confined to the study site from the morning of Day -1 until 48 hours after the drug administration and will be discharged from the site in the morning of Day 3. All other study visits will be ambulatory visits.

**Part 2:**

Part 2 will consist of an up to 41-day screening period, a baseline visit (Day -1), a treatment period of 3 days, followed by a follow-up period (21 days) and a Study Completion visit.

Subjects who undergo both Part 1 and Part 2 have to undergo a wash-out period of six weeks prior to continue in Part 2 with the Baseline visit (i.e. minimum of six weeks between drug administration in part 1 and first drug administration in Part 2).

Eligibility will be assessed at screening and first baseline visit for any study subject including replacement subjects.

All baseline safety evaluation results must be available prior to dosing.

Subjects will be admitted to the study site in the morning of the day prior to dosing (Day - 1) for baseline evaluations. The first day of dosing will occur on Day 1 when 0.25 mg siponimod will be administered. The same dose of 0.25 mg will be administered on Day 2, followed by 0.5 mg on Day 3. Following the last dosing on Day 3, pharmacokinetic assessments will be made for up to 24 hours post dose. Safety assessments will be made for up to 48 hours post dose.

Subjects will be confined to the study site from the morning of Day -1 until 48 hours after the last drug administration and will be discharged from the site in the morning of Day 5. Subjects will undergo End of Study visit starting from Day 25 up to Day 31 (visit window: up to + 6 days).

Safety assessments will include physical examinations, vital signs, standard clinical laboratory evaluations (hematology including lymphocyte, blood chemistry, and urinalysis), 12-Lead ECG, on-line cardiac monitoring, Holter-ECG recording, adverse event, and serious adverse event monitoring.

## Claims

**1.** A method of assessing the appropriate therapeutic dose of 1-{4-[1-(4-cyclohexyl-3-trifluoromethyl-benzyloxyimino)-ethyl]-2-ethyl-benzyl}-azetidine-3-carboxylic acid to administer to a patient in need thereof, comprising the steps of:

(i) testing whether or not the patient has the poor metabolizer genotype; and
(ii) if the patient does not have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose; or
(iii) if the patient does have the poor metaboliser genotype, either

(a) administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at a therapeutic dose below that of the standard therapeutic dose; or
(b) not administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient.

**2.** A method of treating a patient in need with 1-{4-[1-(4-cyclohexyl-3-trifluoromethyl-benzyloxyimino)-ethyl]-2-ethyl-benzyl}-azetidine-3-carboxylic acid, comprising the steps of:

(i) testing whether or not the patient has the poor metabolizer genotype; and
(ii) if the patient does not have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose; or
(iii) if the patient does have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose in combination with a CYP2C9 metabolic activity promoter.

**3.** A method of treating a patient in need with 1-{4-[1-(4-cyclohexyl-3-trifluoromethyl-benzyloxyimino)-ethyl]-2-ethylbenzyl}-azetidine-3-carboxylic acid, or a pharmaceutically acceptable salt thereof, comprising the steps of:

(i) testing whether or not the patient has the poor metabolizer genotype; and
(ii) if the patient does not have the poor metaboliser genotype, administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient at the standard therapeutic dose with a certain level of patient monitoring by a medical practitioner;
or
(iii) if the patient does have the poor metaboliser genotype, administering 1-{4-[1-(4-cyclohexyl-3-trifluoromethylbenzyloxyimino)-ethyl]-2-ethyl-benzyl}-azetidine-3-carboxylic acid, or a pharmaceutically acceptable salt thereof, to the patient at the standard therapeutic dose, with an increased level of patient monitoring by a medical practitioner.

**4.** A method of initiating use of a drug recommended not to be used with 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, comprising the steps of:

(i) testing whether or not the patient has the poor metaboliser genotype; and
(ii) if the patient does not have the poor metaboliser genotype, inititiating use of the drug within a set period after the last dose of 1-{4-[1-(4-cyclohexyl-3-trifluoromethyl-benzyloxyimino)-ethyl]-2-ethyl-benzyl}-azetidine-3-carboxylic acid or a pharmaceutically acceptable salt thereof, has been administered to the patient; or
(iii) if the patient does have the poor metaboliser genotype genotype, inititiating use of the drug within a period longer than the set period of step (ii) above after the last dose of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, has been administered to the patient.

**5.** The method of any one of claims 1 to 4, wherein the poor metabolizer genotype is the CYP2C9*3*3 and CYP2C9*2*3 genotypes.

**6.** The method of claim 5, wherein the the poor metaboliser genotype is the CYP2C9*3*3 genotype.

**7.** The method of any one of claims 1 to 3 or 5-6, wherein the daily standard therapeutic dose is in the range from 1.5 to 2.5 mg.

**8.** The method of claim 7, wherein the daily standard therapeutic dose is 2mg.

**9.** The method according to any one of claims 1 to 8, wherein the patient is suffering from multiple sclerosis.

**10.** The method according to claim 9, wherein the multiple sclerosis is secondary progressive multiple sclerosis with relapses (rSPMS) or secondary progressive multiple sclerosis without relapses (SPMS).

**11.** The method according to claim 4, wherein the set period of step (ii) is in the range from 3-14 days.

**12.** A method for the treatment of an autoimmune condition in a patient in need thereof, said patient having the poor metabolizer genotype, comprising administering 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, to the patient in a daily amount in the range from 0.25 - 0.75mg.

**13.** The method of claim 12, wherein the poor metaboliser genotype is the CYP2C9*3*3 genotype.

**14.** The method of claim 12 or 13, wherein the daily amount is a therapeutic dosage of 0.5mg.

**15.** The method of any one of claims 12 to 14, wherein the autoimmune condition is multiple sclerosis.

**16.** The method of claim 15, wherein the multiple sclerosis is multiple sclerosis with relapses (rSPMS) or secondary progressive multiple sclerosis without relapses (SPMS).

**17.** A pharmaceutical combination comprising (a) 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof; and (b) a CYP2C9 metabolic activity promoter for simultaneous, separate or sequential use.

**18.** A method of optimizing the daily dosage of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof to patient in need, said method comprising the steps of:

(i) measuring the patient blood lymphocyte level following administration of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof at a dose of about 2mg per day; and
(ii) in cases where the blood lymphocyte level is reduced below a level of 0.2x10e9/L following daily administration of 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof at the therapeutic dosage, reducing the the daily dosage to about 1mg per day.

**19.** 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, for use in a method of treating an autoimmune disease, said method being according to any of claims 1-18.

**20.** 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof, in the manufacture of a medicament for the treatment of an autoimmune disease, said method being according to any of claims 1-18.

**Figure 1-1 Time-dependent biotransformation of [$^{14}$C]BAF312**

The kinetics of the biotransformation of 1 and 10 µM [$^{14}$C]BAF312 was investigated using human liver microsomes (0.3 mg protein/mL). The disappearance of [$^{14}$C]BAF312 was determined by HPLC analysis combined with radiodetection.

1 µM [$^{14}$C]BAF312

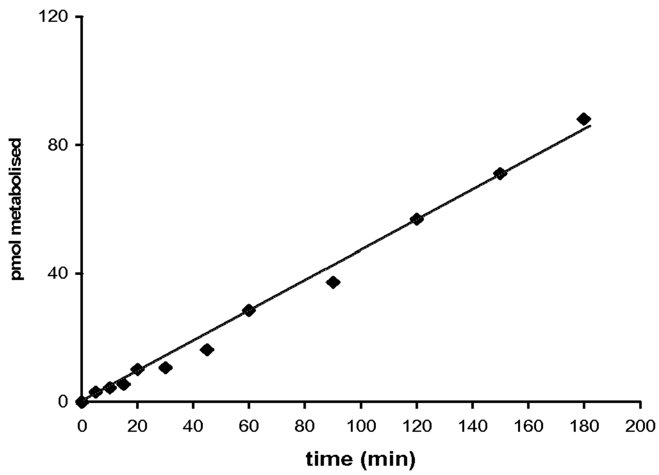


10 µM [$^{14}$C]BAF312

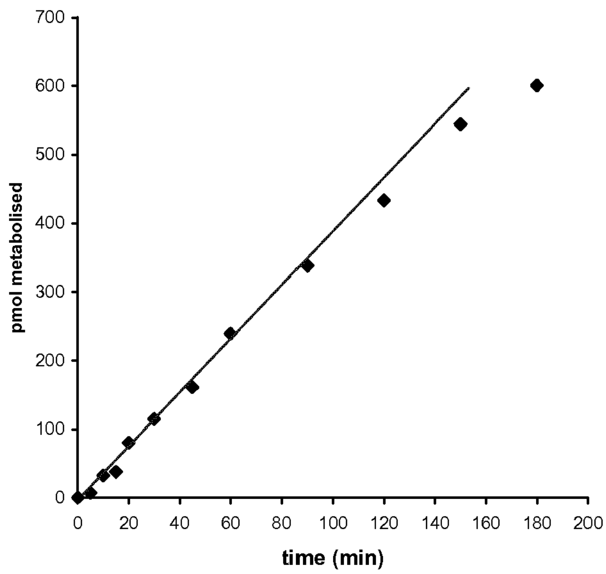

**Figure 1-2 Protein-dependent biotransformation of [$^{14}$C]BAF312**

The protein-dependence of 10 µM [$^{14}$C]BAF312 metabolism was investigated (60 minutes incubations) using human liver microsomes. The disappearance of [$^{14}$C]BAF312 was determined by HPLC analysis combined with radiodetection.

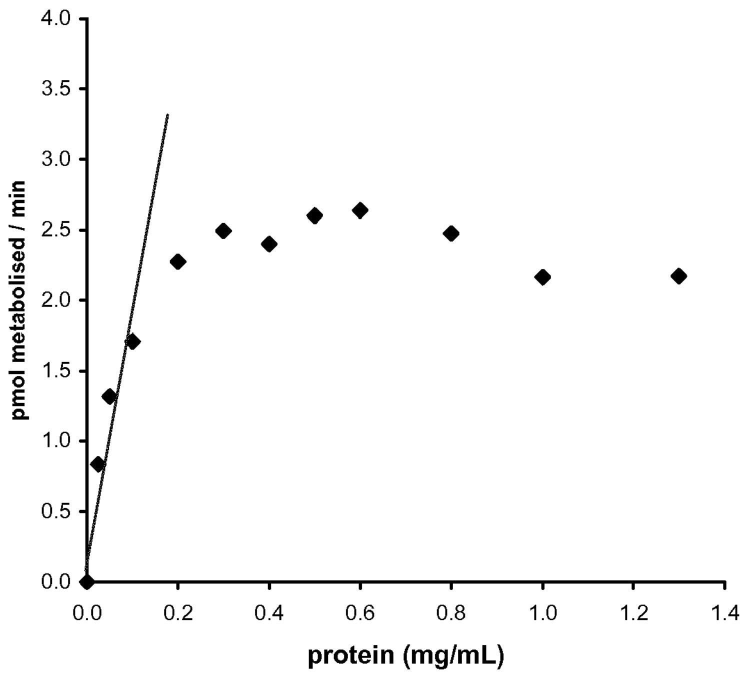

**Figure 1-3 Enzyme kinetics of [$^{14}$C]BAF312 biotransformation in human liver microsomes**

The concentration-dependent kinetics of [$^{14}$C]BAF312 biotransformation in pooled human liver microsomes (0.1 mg/mL) after 90 minutes incubation was plotted as Michaelis-Menten plot (upper) and as Eadie-Hofstee plot (bottom) fitted with substrate inhibition model.

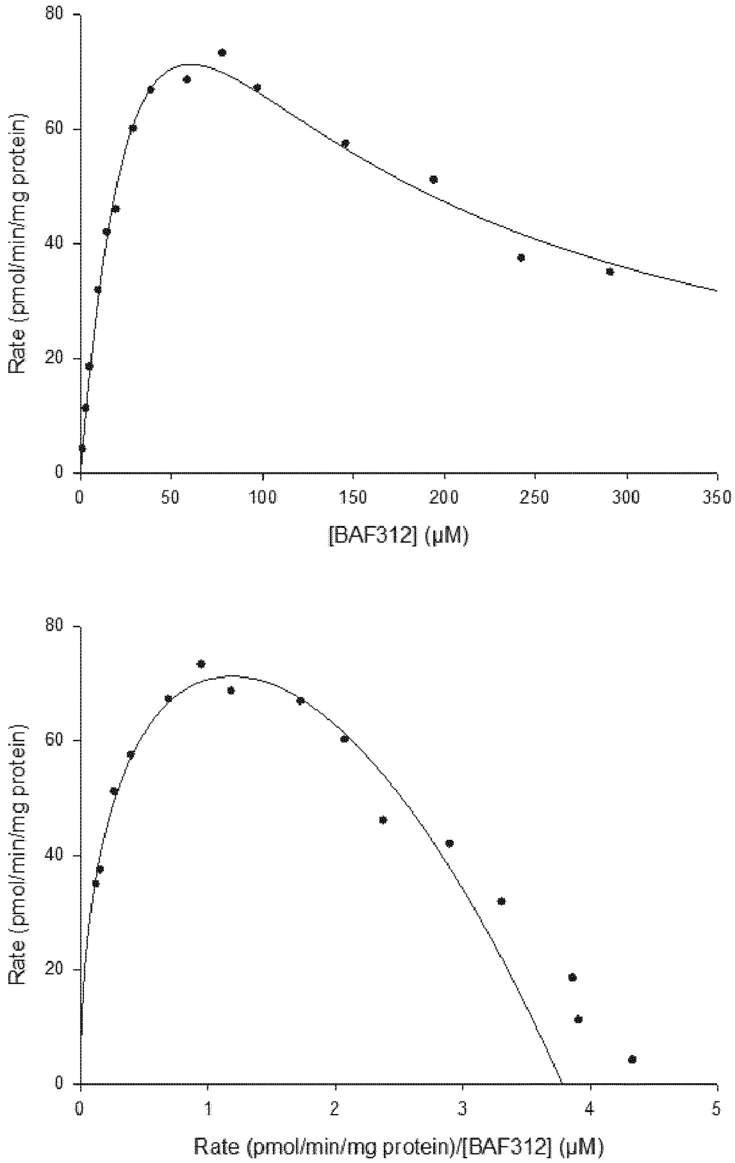


| Kinetic model | $V_{max}$ [pmol/min/mg] | $K_m^{app}$ [μM] | $V_{max}/K_m$ μL/mg/min |
|---|---|---|---|
| Substrate Inhibition (uncompetitive) | 191 ± 25 | 50.3 ± 9.7 | 3.8 |

**Figure 1-4 Biotransformation of [$^{14}$C]BAF312 by recombinant human CYP P450s**

The biotransformation of [$^{14}$C]BAF312 by recombinant enzymes (30 pmol/mL) in insect cell membranes expressing a single human cytochrome P450 isoenzyme and by HLM (108 pmol CYP/mL) was investigated after 30 minutes incubations at 10 and 40 µM. The formation of metabolites was determined by HPLC analysis combined with radiodetection.

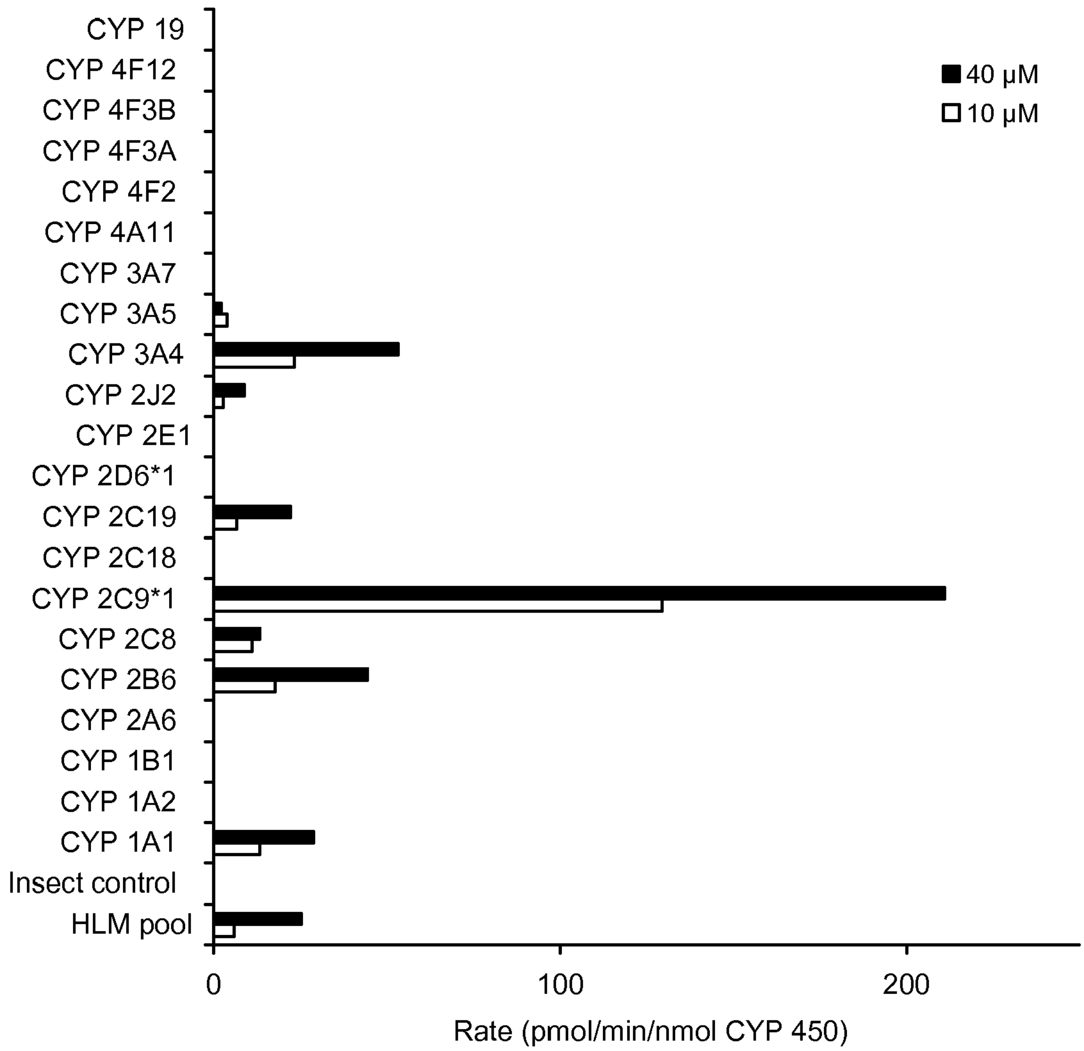

**Figure 1-5 Enzyme kinetics of [$^{14}$C]BAF312 metabolism by rhCYP2C9**

The concentration-dependent kinetics of [$^{14}$C]BAF312 biotransformation (total metabolites) in rhCYP2C9 (50 pmol/mL) after 60 minutes incubation was plotted as Michaelis-Menten plot (upper) and as Eadie-Hofstee plot (V vs. V/S, bottom) fitted with substrate inhibition model using substrate concentrations 5-300 µM.

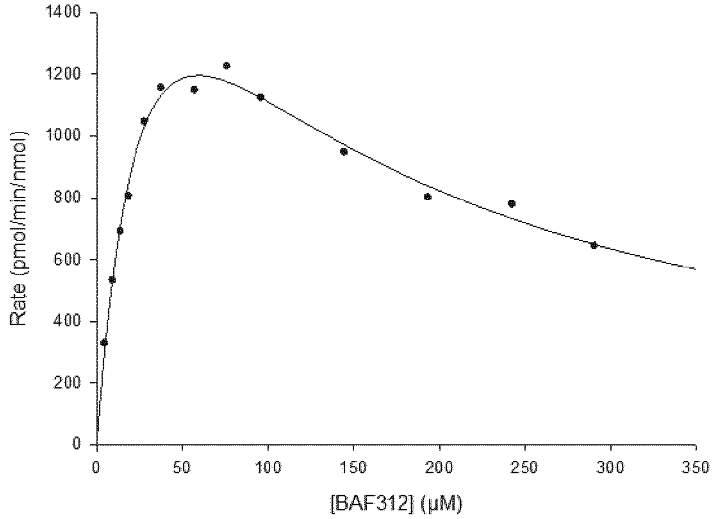


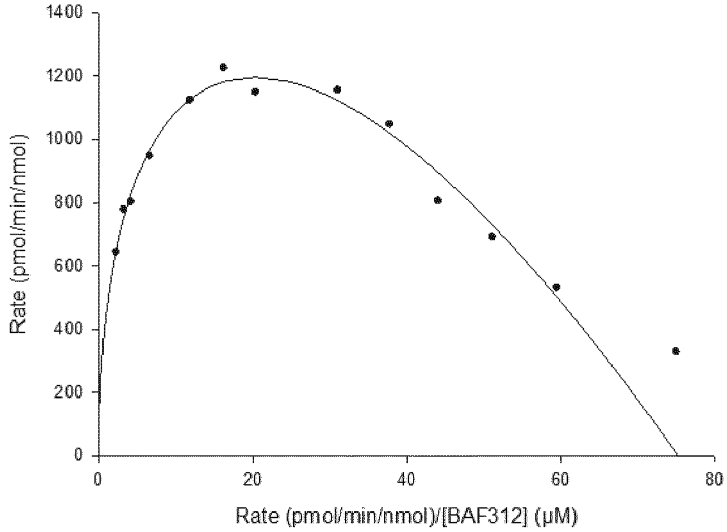


| $V_{max}$ [pmol/min/nmol] | $K_m^{app}$ [µM] | $V_{max}/K_m$ µL/nmol/min |
|---|---|---|
| 2596 ± 233 | 34.5 ± 5.0 | 75.2 |

**Figure 1-6 Enzyme kinetics of [$^{14}$C]BAF312 metabolism by rhCYP3A4**

The concentration-dependent kinetics of [$^{14}$C]BAF312 biotransformation (total metabolites) in rhCYP3A4 (50 pmol/mL) after 30 minutes incubation was plotted as Michaelis-Menten plot (upper) and as Eadie-Hofstee plot (V vs. V/S, bottom) using substrate concentrations 5-250 µM.

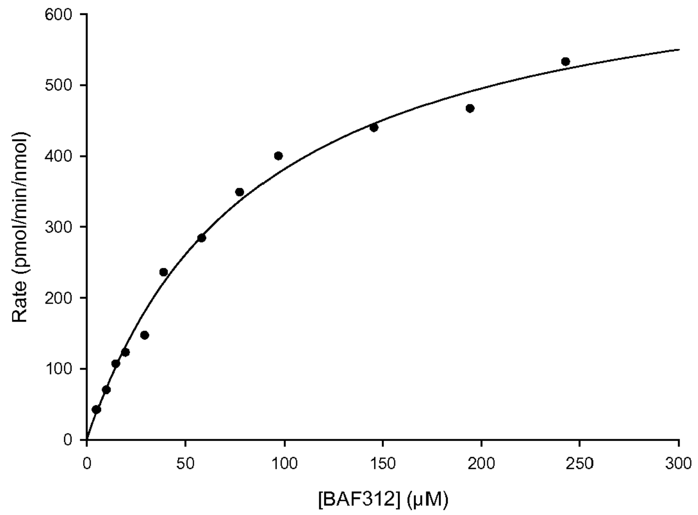


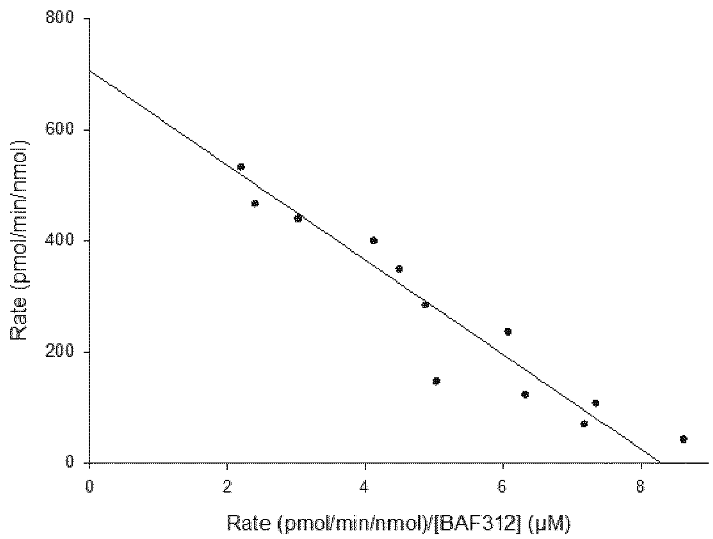


| $V_{max}$ [pmol/min/nmol] | $K_m^{app}$ [µM] | $V_{max}/K_m$ µL/nmol/min |
|---|---|---|
| 706 ± 31 | 85.1 ± 8.6 | 8.30 |

**Figure 1-7 Inhibition of BAF312 metabolism in HLM by chemical inhibitors**

Biotransformation of [$^{14}$C]BAF312 (5 µmol/L) by human liver microsomes (0.1 mg/mL) was investigated in the presence and absence of different inhibitors. The supernatants of incubations after 90 minutes were analyzed by HPLC with radiodetection.

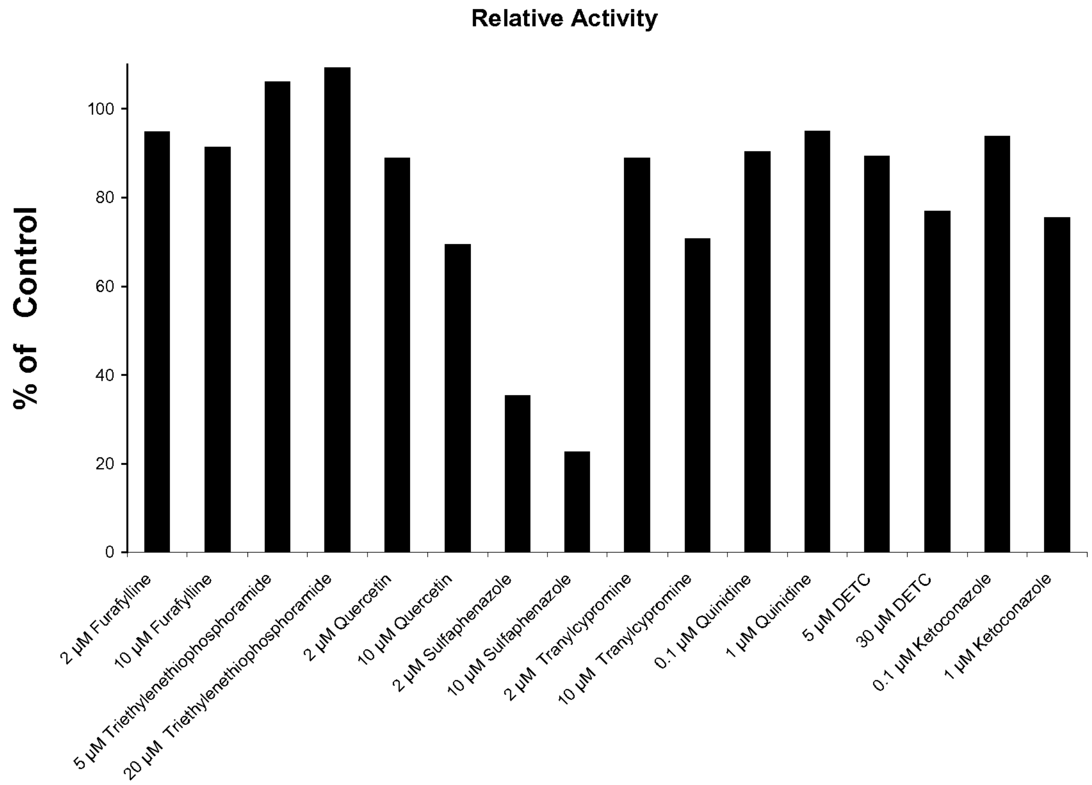

**Figure 1-8 Comparison of [$^{14}$C]BAF312 metabolism rates in HLM from individual donors with 3 different CYP2C9 genotypes (Data from Table 1-5)**

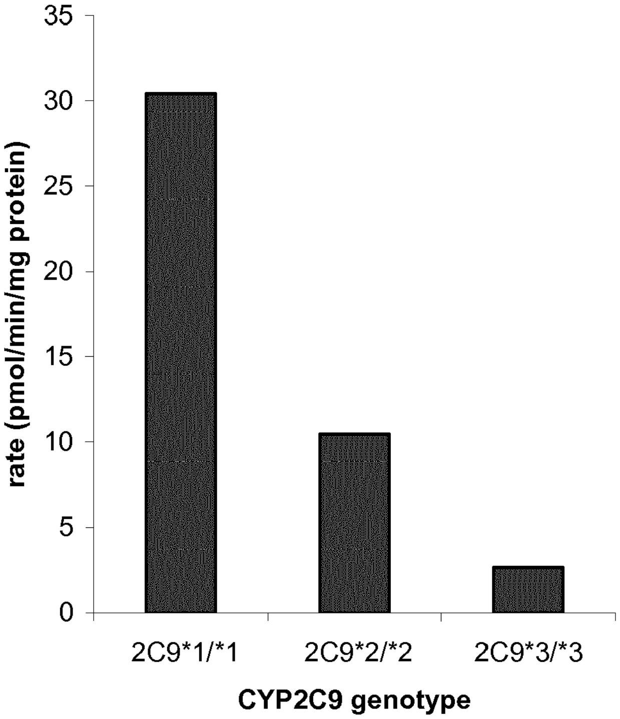

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010072703 A


### Non-patent literature cited in the description


- **ROWLAND YEO K ; ROSTAMI-HODJEGAN A ; TRUCKER GT.** Abundance of cytochromes P450 in human liver: a meta-analysis. *Br. J. Clinical Pharmacology,* 2004, vol. 57, 687-688
- **BJORNSSON TD ; CALLAGHAN JT ; EINOLF HJ ; FISCHER V ; GAN L ; GRIMM S ; KAO J ; KING SP ; MIWA G ; NI L.** The conduct of in vitro and in vivo drug-drug interaction studies: A Pharmaceutical Research and Manufacturers of America (PhRMA) perspective. *Drug Metab Dispos,* 2003, vol. 31, 815-832
- **GUENGERICH FP.** In vitro techniques for studying drug metabolism. *Journal of Pharmacokinetics & Biopharmaceutics,* 1996, vol. 24, 521-533
- **NEWTON DJ ; WANG R W ; LU AYH.** Cytochrome P450 inhibitors: Evaluation of specificities in the in vitro metabolism of therapeutic agents by human liver microsomes. *Drug Metabolism and disposition,* 1995, vol. 25, 154-158
- In vitro approaches for studying the inhibition of drug-metabolizing enzymes and identifying the drug-metabolizing enzymes responsible for the metabolism of drugs (Reaction phenotyping ) with emphasis on cytochrome P450. **OGILVIE BW ; USUKI E ; YERINO P ; PARKINSON A.** Drug-drug Interactions. Informa Healthcare, 2008, 231-358
- **RETTIE AE ; JONES JP GT.** Clinical and toxicological relevance of CYP2C9: drug-drug interactions and pharmacogenetics. *Annu. Rev. Pharmacol,* 2005, vol. 45, 477-494
- **SHIMADA T ; YAMAZAKI H ; MIMURA M et al.** Interindividual variations in human liver cytochrome P-450 enzymes involved in the oxidation of drugs, carcinogens and toxic chemicals: studies with liver microsomes of 30 Japanese and 30 Caucasians. *JPET,* 1994, vol. 270, 414-423
- **TUCKER GT ; HOUSTON JB ; HUANG SM.** Optimizing drug development: strategies to assess drug metabolism/transporter interaction potential-towards a consensus. *Br J Clin Pharmacol,* 2001, vol. 52, 107-17